# EUROPEAN PATENT APPLICATION

(11) **EP 3 272 747 A1**
(43) Date of publication of application: **24.01.2018**
(21) Application number: 16764171.1
(22) Date of filing: 02.03.2016
(51) Int. Cl.: C07D 403/14

(54) **METHOD OF PREPARATION FOR LEDIPASVIR AND DERIVATIVE THEREOF, AND INTERMEDIATE COMPOUND FOR PREPARATION OF LEDIPASVIR**

(30) Priority: 17.03.2015 CN 201510117997
(71) Applicant: Shanghai Forefront Pharmceutical Co., Ltd, Shanghai 201203 (CN)
(72) Inventor: HUANG, Chengjun, Shanghai 201203 (CN); FU, Gang, Shanghai 201203 (CN); FU, Shaojun, Shanghai 201203 (CN); WEI, Zhewen, Shanghai 201203 (CN); LI, Wei, Shanghai 201203 (CN); ZHANG, Xixuan, Shanghai 201203 (CN)
(74) Representative: Predazzi, Valentina
(86) International application number: PCT/CN2016/075358
(87) International publication number: WO 2016/145990

(57) **Abstract**

The present invention relates to methods of preparation for Ledipasvir and derivatives thereof and intermediate compounds for preparation of Ledipasvir. Specifically, the invention discloses a method for preparing the compounds of formula 1 and a series of new preparation methods of preparing Ledipasvir. The method of the invention is simple and efficient, and has good application prospect.

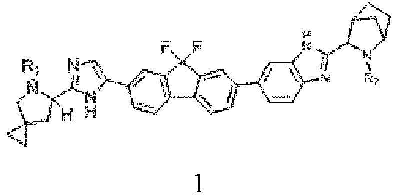

## Description

### Technical Field

The present invention belongs to the field of pharmaceutical synthesis. Specifically, the present invention relates to a method of preparation for Ledipasvir and derivatives thereof and intermediate compounds for preparation of Ledipasvir; and more specifically, relates to a method for preparing the compounds of formula 1.

### Background

Ledipasvir (LDV, the structure is as shown in formula 1-LDV) is a therapeutic drug for hepatitis C developed by Gilead. FDA has approved the breakthrough therapy LDV/SOF (Sofosbuvir) fixed-dose combination drug, and the combination therapy is expected to cure genotype 1 HCV patients in a period of short to 8 weeks without iniection of interferon or combination with ribavirin.

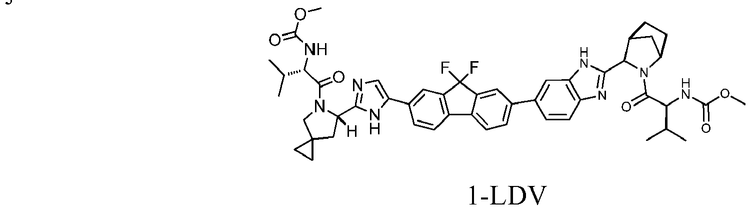

US20100310512 has reported a synthetic route of Ledipasvir as follows:

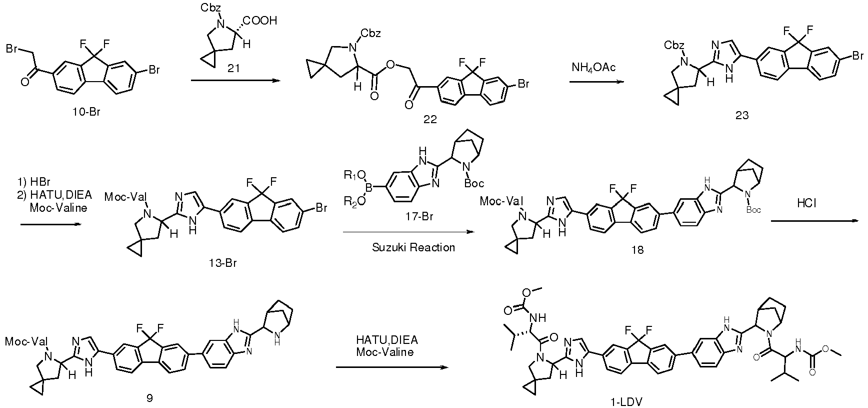

The two side chains of compound 1-LDV are both Moc-Val, but in compound 21 Cbz- is first introduced, then in compound 13-Br Moc-Val on the left is introduced by hydrolysis and condensation, and as for the right side chain, Boc- is first introduced in 17-Br and then Moc-Val on the right is introduced by hydrolysis and condensation, which means the target product was obtained by introduction of protecting group firstly and then by two times of hydrolysis as well as condensation. The reaction process is tedious and the raw materials are expensive. The tedious synthesis method makes the raw material cost more expensive, so it is needed to use more efficient way to reduce the cost of raw materials.

US2013324740 has reported a preparation method for Ledipasvir as follows:

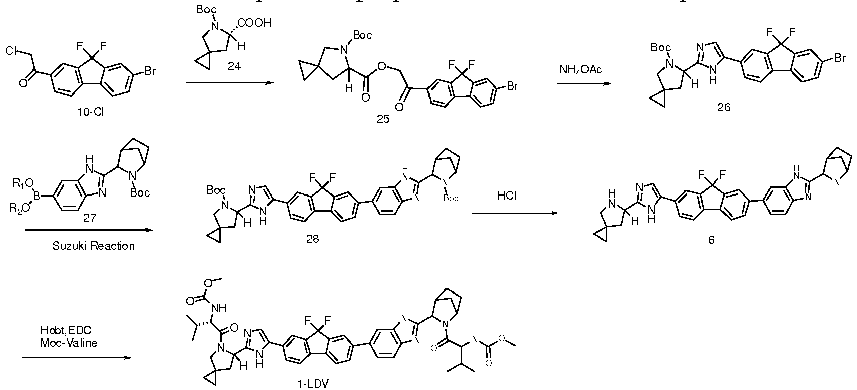

This method is more efficient than that of US20100310512, but it still requires the preparation of Boc-protected compounds 24 and 27, which need to be deprotected by hydrolysis, and the yield is still not high, and the emission of three wastes will be also increased.

Therefore, it is still needed to find a more convenient and efficient preparation method for Ledipasvir.

### Summary of invention

The object of the present invention is to provide a series of methods for preparing Ledipasvir.

Another object of the present invention is to provide a method for preparing Ledipasvir derivatives.

Another object of the present invention is to provide a series of intermediates for preparing Ledipasvir or derivatives thereof.

In the first aspect of the present invention, a method for preparing a compound of formula 1 is provided, 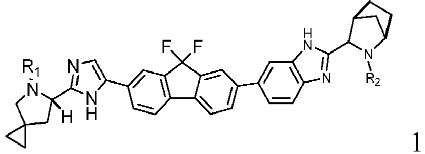 which comprises steps of:
subjecting a compound of formula 2-1 with a compound of formula 3-1 to coupling reaction to give a compound of formula 1; 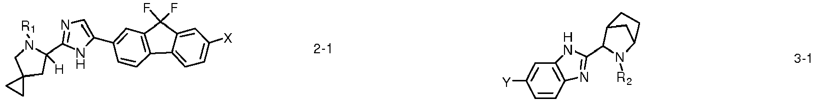 wherein,
   R₁ and R₂ are defined as follows:
   when R₁ is benzyloxycarbonyl (Cbz), R₂ is benzyloxycarbonyl (Cbz), t-butoxycarbonyl (Boc) or (S)-2-methoxycarbonylamino-3-methyl-butyryl (Moc-Val); or
   when R₁ is t-butoxycarbonyl (Boc), R₂ is benzyloxycarbonyl(Cbz) or (S)-2-methoxycarbonylamino-3-methyl-butyryl (Moc-Val); or
   when R₁ is (S)-2-methoxycarbonylamino-3-methyl-butyryl(Moc-Val), R₂ is benzyloxycarbonyl (Cbz) or (S)-2-methoxycarbonylamino-3-methyl-butyryl (Moc-Val);
   X and Y are defined as follows:
      when X is halogen (Cl, Br or I), Y is -B(OR₃)(OR₄) or -BF₃K; or
      when X is -B(OR₃)(OR₄) or -BF₃K, Y is halogen (Cl, Br or I), wherein R₃ and R₄ are independently H or C 1-C6 linear or branched alkyl, or two of them together form C2-C8 linear or branched alkylidene;
      or which comprises steps of:
         (i) subjecting a compound of formula 2-2 to acylation reaction to give a compound of formula 4; 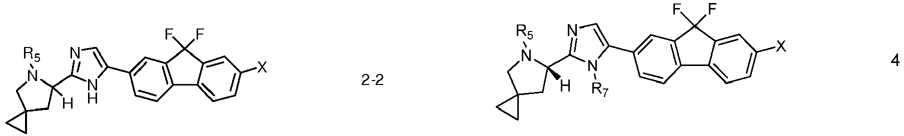 and subjecting a compound of formula 3-2 to acylation reaction to obtain a compound of formula 5; 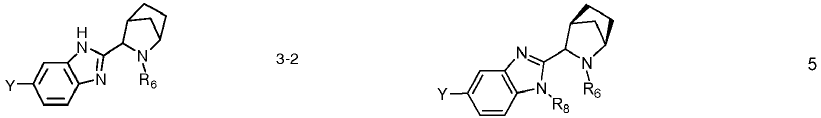
         (ii) subjecting the compound of formula 4 with the compound of formula 5 to coupling reaction to obtain a mixture of a compound of formula 1' and a compound of formula 1; 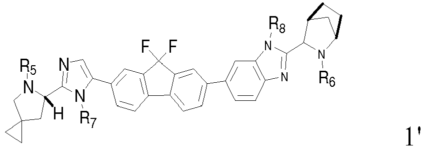
         (iii) subjecting the mixture of a compound of formula 1' and a compound of formula 1 to hydrogenation reduction or hydrolysis reaction to obtain a compound of formula 1;
      in each of the above scheme,
      X and Y are defined as follows:
         when X is halogen (Cl, Br or I), Y is -B(OR₃)(OR₄) or -BF₃K; or
         when X is -B(OR₃)(OR₄) or -BF₃K, Y is halogen (Cl, Br or I), wherein R₃ and R₄ are independently H or C 1-C6 linear or branched alkyl, or two of them together form C2-C8 linear or branched alkylidene;
         R₅ and R₆ are independently benzyloxycarbonyl (Cbz), t-butoxycarbonyl (Boc) or (S)-2-methoxycarbonylamino-3-methyl-butyryl (Moc-Val);
         R₇ and R₈ are independently C1-C5 acyl, Cbz, Boc, or 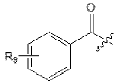 wherein, R₉ is a substituent at any position of benzene ring (ortho-, meta- or para-positionion), and the substituent is C1-C4 alkyl or halogen.

In another preferred embodiment, the method has one or more features selected from the group consisting of:
the coupling reaction is carried out in an inert solvent (such as THF, 2-methyl THF, dioxane, ether (C₁-C₄OC₁-C₄), C₁-C₅ alcohol or ester (C₁-C₄COOC₁-C₄));
the coupling reaction is carried out in the presence of Pd(0) or Pd(II) catalysts such as Pd(PPh₃)₄, PdCl₂(PPh₃)₂, Pd(OAc)₂, PdCl₂(P(t-Bu)₂Ph)₂, Pd(dppf)₂Cl₂ or its methylene dichloride complex;
the hydrogenation reaction is carried out in the presence of Pt, Pd or Ni catalyst;
the hydrolysis reaction is carried out in the presence of proton acid and / or Lewis acid.

In another preferred embodiment, the proton acid and Lewis acid are selected from the group consisting of HCl, H₂SO₄, CF₃SO₄, CH₃SO₄ or combinations thereof.

In the second aspect of the present invention, a method for preparing a compound of formula 1-LDV is provided, 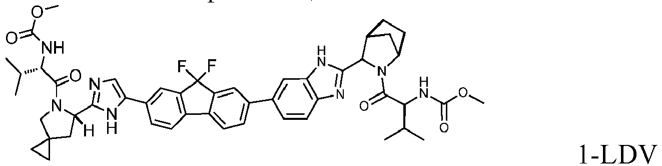 which comprises steps of:
(i) subjecting a compound of formula 1 to hydrogenation reduction or hydrolysis to obtain a compound of formula 6; 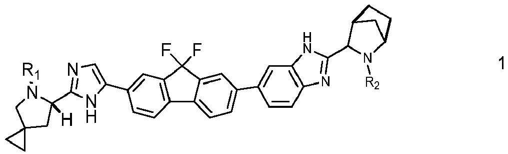 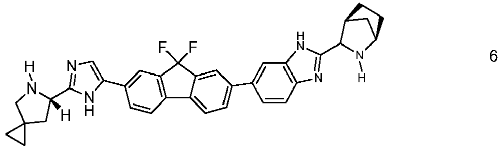 wherein, both of R₁ and R₂ are Cbz;
(ii) reacting the compound of formula 6 with (S)-2-methoxycarbonylamino-3-methyl-butyric acid to produce a compound of formula 1-LDV;
or which comprises steps of:
(i) subjecting a compound of formula 1 to hydrogenation reduction or hydrolysis to obtain a compound of formula 7; 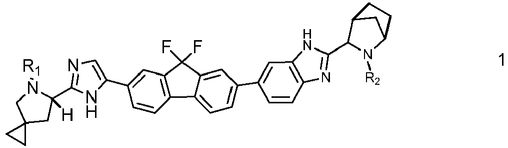 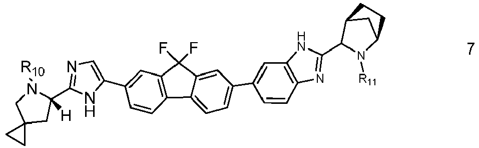 wherein,
   when in compound 1 R₁ is Cbz and R₂ is Boc, in compound 7 R₁₀ is H and R₁₁ is Boc or H; or
   when in compound 1 R₁ is Boc and R₂ is Cbz, in compound 7 R₁₀ is Boc or H and R₁₁ is H;
(ii) subjecting the compound of formula 7 to hydrolysis reaction to give a compound of formula 6; 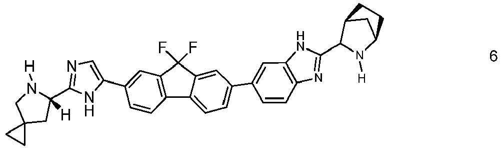
(iii) reacting the compound of formula 6 with (S)-2-methoxycarbonylamino-3-methyl-butyric acid to produce a compound of formula 1-LDV;
or which comprises steps of:
(i) subjecting a compound of formula 1 to hydrogenation reduction or hydrolysis to obtain a compound of formula 8; 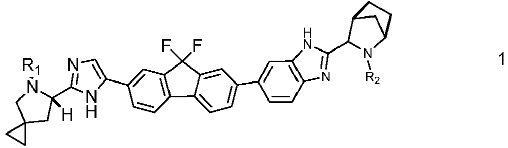 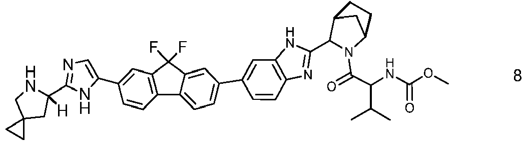 wherein, R₁ is Cbz or Boc; R₂ is Moc-Val;
(ii) reacting the compound of formula 8 with (S)-2-methoxycarbonylamino-3-methyl-butyric acid to produce a compound of formula 1-LDV;
or which comprises steps of:
(i) subjecting a compound of formula 1 to hydrogenation reduction or hydrolysis to obtain a compound of formula 9; 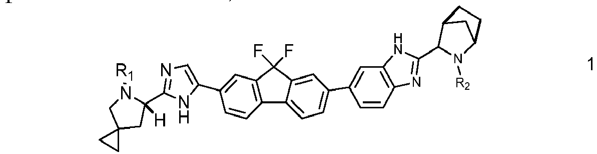 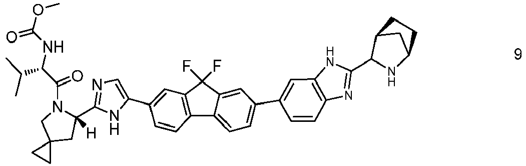 wherein, R₁ is Moc-Val; R₂ is Cbz.
(ii) reacting the compound of formula 9 with (S)-2-methoxycarbonylamino-3-methyl-butyric acid to produce a compound of formula 1-LDV;

In another preferred embodiment, the method has one or more features selected from the following group:
the hydrogenation reaction is carried out in the presence of Pt, Pd or Ni catalyst;
the hydrolysis reaction is carried out in the presence of proton acid and / or Lewis acid.

In another preferred embodiment, the proton acid, Lewis acid are selected from the group consisting of HCl, H₂SO₄, CF₃SO₄, CH₃SO₄ or combinations thereof.

In the third aspect of the present invention, a method for preparing a compound of formula 1-LDV is provided, 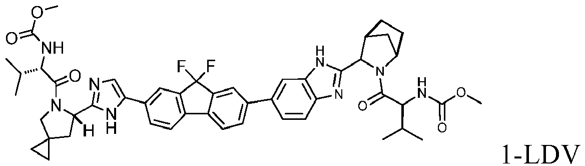 which comprises steps of:
(i) reacting a compound of formula 10 with a compound of formula 11 to obtain a compound of formula 12; 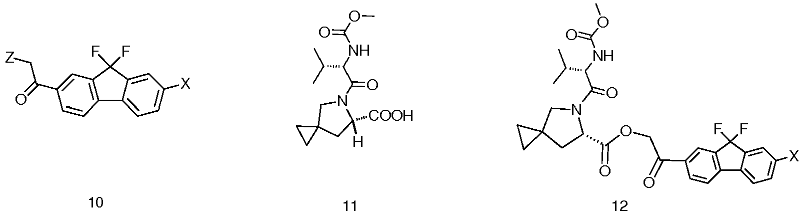
(ii) subjecting the compound of formula 12 with an ammonium salt to cyclization reaction to give a compound of formula 13; 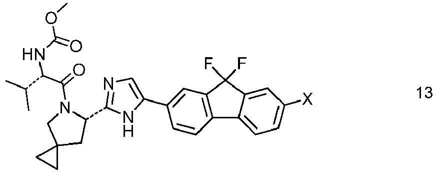
(iii) subjecting the compound of formula 13 with a compound of formula 14 to coupling reaction to give a compound of formula 1-LDV; 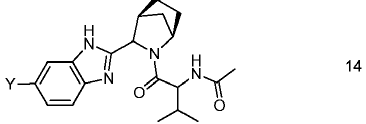 wherein,
   when X is halogen (Cl, Br or I) then Y is -B(OR₃)(OR₄) or -BF₃K; or when X is -B(OR₃)(OR₄) or -BF₃K, then Y is halogen (Cl, Br or I), wherein R₃ and R₄ are independently H or C1-C6 linear or branched alkyl, or two of them together form C2-C8 linear or branched alkylidene;
   Z is halogen (chlorine, bromine or iodine);
   or which comprises steps of:
   (i) reacting a compound of formula 10 with a compound of formula 11 to obtain a compound of formula 12; 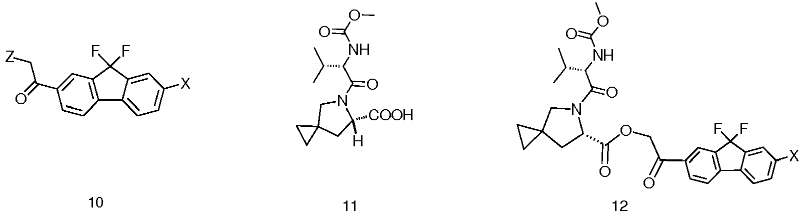
   (ii) subjecting the compound of formula 12 with an ammonium salt to cyclization reaction to give a compound of formula 13; 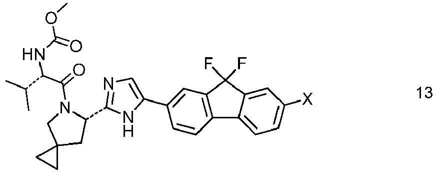
   (iii) subjecting the compound of formula 13 to acylation reaction to produce a compound of formula 15; and 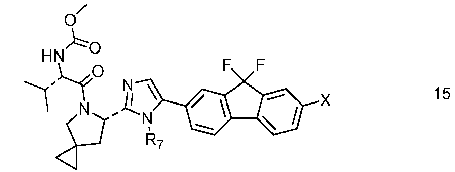 subjecting a compound of formula 14 to acylation reaction to obtain a compound of formula 16; 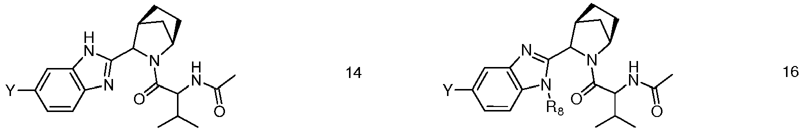
   (iv) subjecting the compound of formula 15 with the compound of formula 16 to coupling reaction to obtain a mixture of a compound of formula 1'-LDV and a compound of formula 1-LDV; 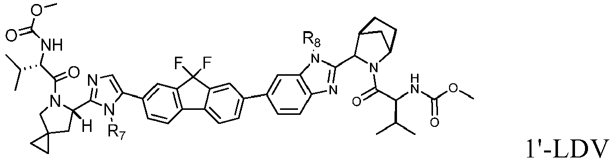
   (v) subjecting the mixture of a compound of formula 1'-LDV and a compound of formula 1-LDV to hydrogenation reduction or hydrolysis to obtain a compound of formula 1-LDV; wherein,
      when X is halogen (Cl, Br or I), then Y is -B(OR₃)(OR₄) or -BF₃K; or when X is -B(OR₃)(OR₄) or -BF₃K, then Y is halogen (Cl, Br or I); wherein R₃ and R₄ are independently H or C1-C6 linear or branched alkyl, or two of them together form C2-C8 linear or branched alkylidene;
      R₇ and R₈ are independently C₁-C₅ acyl, Cbz, Boc, or 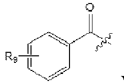 wherein, R₉ is a substituent at any position of benzene ring (ortho-, meta- or para-positionion), and the substituent is C1-C4 alkyl or halogen.

In another preferred embodiment, the method has one or more features selected from the following group:
the coupling reaction is carried out in an inert solvent (such as THF, 2-methyl THF, dioxane, ether (C₁-C₄OC₁-C₄), C₁-C₅ alcohol or ester (C₁-C₄COOC₁-C₄));
the coupling reaction is carried out in the presence of Pd(0) or Pd(II) catalysts (such as Pd(PPh₃)₄, PdCl₂(PPh₃)₂, Pd(OAc)₂, PdCl₂(P(t-Bu)₂Ph)₂, Pd(dppf)₂Cl₂ or its methylene dichloride complex);
the hydrogenation reaction is carried out in the presence of Pt, Pd or Ni catalyst;
the hydrolysis reaction is carried out in the presence of proton acid and / or Lewis acid;
in the cyclization reaction the ammonium salt is selected from ammonium acetate, ammonium chloride, ammonium sulfate, ammonium phosphate, ammonium carbonate or ammonium hydrogen carbonate.

In another preferred embodiment, the proton acid, Lewis acid are selected from the group consisting of HCl, H₂SO₄, CF₃SO₄, CH₃SO₄ or combinations thereof.

In the fourth aspect of the present invention, a method for preparing a compound of formula 1-LDV is provided, 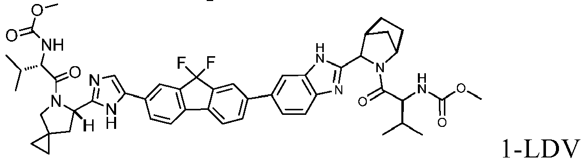 which comprises steps of:
(i) reacting a compound of formula 10 with a compound of formula 11 to obtain a compound of formula 12; 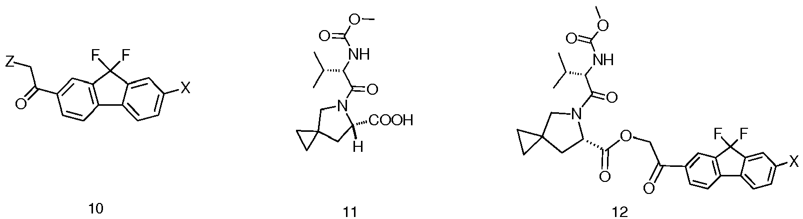
(ii) subjecting the compound of formula 12 with an ammonium salt to cyclization reaction to give a compound of formula 13; 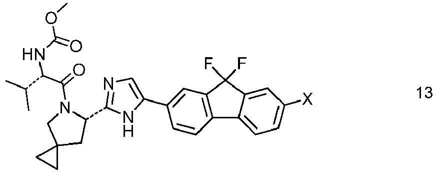
(iii) subjecting the compound of formula 13 with a compound of formula 17 to coupling reaction to give a compound of formula 18; 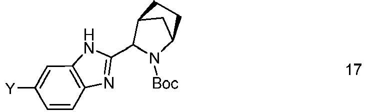 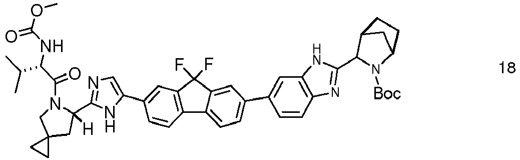
(iv) subjecting the compound of formula 18 to hydrolysis reaction to give a compound of formula 9; 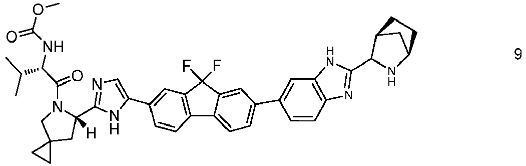
(ii) reacting the compound of formula 9 with (S)-2-methoxycarbonylamino-3-methyl-butyric acid to produce a compound of formula 1-LDV;
   wherein,
   when X is halogen (Cl, Br or I), then Y is -B(OR₃)(OR₄) or -BF₃K; or when X is -B(OR₃)(OR₄) or -BF₃K, then Y is halogen (Cl, Br or I), wherein R₃ and R₄ are independently H or C1-C6 linear or branched alkyl, or two of them together form C2-C8 linear or branched alkylidene;
   Z is halogen (chlorine, bromine or iodine);
   or which comprises steps of:
   (i) reacting a compound of formula 10 with a compound of formula 11 to obtain a compound of formula 12; 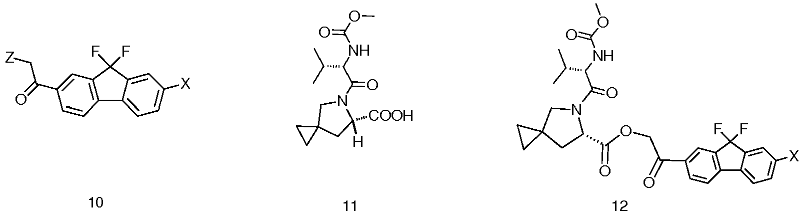
   (ii) subjecting the compound of formula 12 with an ammonium salt to cyclization reaction to give a compound of formula 13; 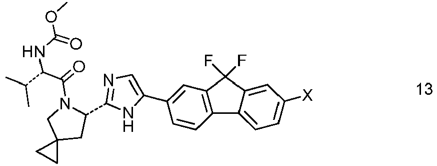
   (iii) subjecting the compound of formula 13 to acylation reaction to produce a compound of formula 15; 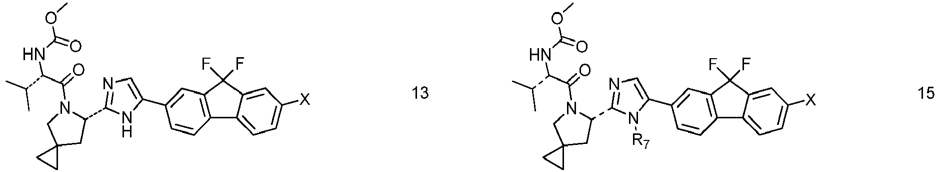 and subjecting a compound of formula 17 to acylation reaction to give a compound of formula 19; 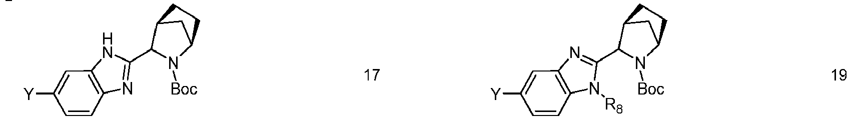
   (iv) subjecting the compound of formula 15 with the compound of formula 19 to coupling reaction to obtain a mixture of a compound of formula 18' and a compound of formula 18; 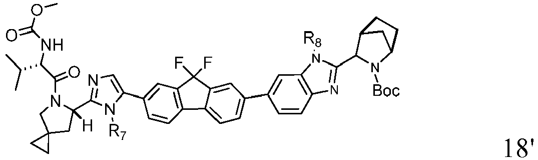 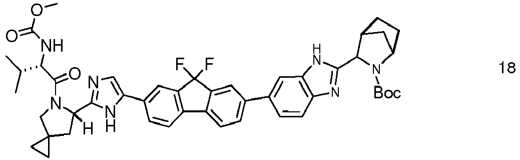
   (v) subjecting the mixture of a compound of formula 18' and a compound of formula 18 to hydrolysis to obtain a compound of formula 9; 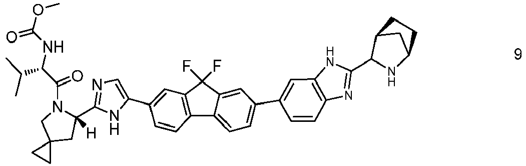
   (iv) reacting the compound of formula 9 with (S)-2-methoxycarbonylamino-3-methyl-butyric acid to produce a compound of formula 1-LDV;
      wherein,
      when X is halogen (Cl, Br or I), then Y is -B(OR₃)(OR₄) or -BF₃K; or when X is -B(OR₃)(OR₄) or -BF₃K, then Y is halogen (Cl, Br or I), wherein R₃ and R₄ are independently H or C1-C6 linear or branched alkyl, or two of them together form C2-C8 linear or branched alkylidene;
      Z is halogen (chlorine, bromine or iodine);
      R₇ and R₈ are independently C1-C5 acyl, Cbz, Boc, or 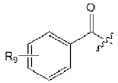 wherein, R₉ is a substituent at any position of benzene ring (ortho-, meta- or para-positionion), and the substituent is C1-C4 alkyl or halogen.

In another preferred embodiment, the method has one or more features selected from the following group:
the coupling reaction is carried out in an inert solvent (such as THF, 2-methyl THF, dioxane, ether (C1-C4OC1-C4), C1-C5 alcohol or ester (C1-C4COOC1-C4));
the coupling reaction is carried out in the presence of Pd(0) or Pd(II) catalysts such as Pd(PPh₃)₄, PdCl₂(PPh₃)₂, Pd(OAc)₂, PdCl₂(P(t-Bu)₂Ph)₂, Pd(dppf)₂Cl₂ or its methylene dichloride complex;
the hydrolysis reaction is carried out in the presence of proton acid and / or Lewis acid.

In another preferred embodiment, the proton acid, Lewis acid are selected from the group consisting of HCl, H₂SO₄, CF₃SO₄, CH₃SO₄ or combinations thereof.

In another preferred embodiment, in the cyclization reaction, the ammonium salt is selected from ammonium acetate, ammonium chloride, ammonium sulfate, ammonium phosphate, ammonium carbonate or ammonium hydrogen carbonate.
in the fifth aspect of the present invention an intermediate compound for preparing Ledipasvir is provided, 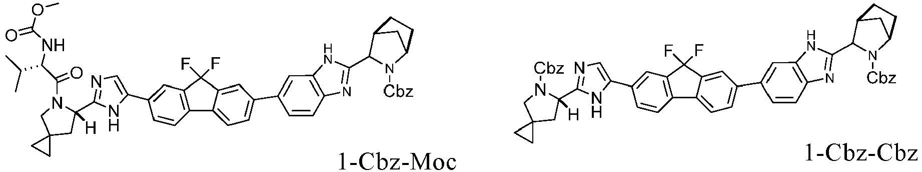 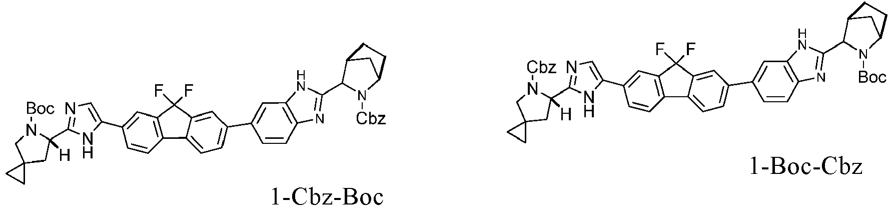 and 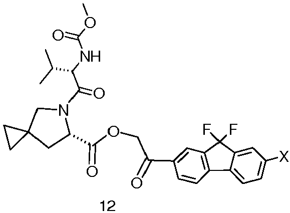 wherein, when X is halogen (Cl, Br or I), then Y is -B(OR₃)(OR₄) or -BF₃K; or when X is -B(OR₃)(OR₄) or -BF₃K, then Y is halogen (Cl, Br or I); wherein R₃ and R₄ are independently H or C1-C6 linear or branched alkyl, or two of them together form C2-C8 linear or branched alkylidene;

It should be understand that within the scope of the invention each of the technical features described in detail above and below (such as the examples) can be combined with each other seperately , so as to form a new or preferred technical proposal, which will no longer be described herein due to the length limitation.

### Brief Description of the Drawings

Figure 1 shows HPLC spectra of the product in Comparative Example 11 which shows the content of defluorinated impurity.
Figure 2 shows HPLC spectra of the product in Comparative Example 12 which shows the content of defluorinated impurity.

### Detailed Description of the Invention

After extensive and deep research the inventors of the present invention have carried out extensive screening and optimization of the preparation method for Ledipasvir and its derivatives. They first disclose several preparation prosess with advantages such as simple preparation, high yield, high purity and less impurity. The present invention also provides novel intermediate compounds for preparing Ledipasvir. The invention is completed on this basis,.

### Preparation method

The invention provides a variety of preparation methods for Ledipasvir, the preparation methods comprise one or more of the following characteristics:
1) the reaction temperature is 0-100 °C, preferably 20-90 °C;
2) the reaction time is 0.1-48 h, preferably 2-20 h;
3) the reaction is carried out in an inert solvent.

The preferred preparation methods of the present invention are shown in route 1-3.

### Route 1

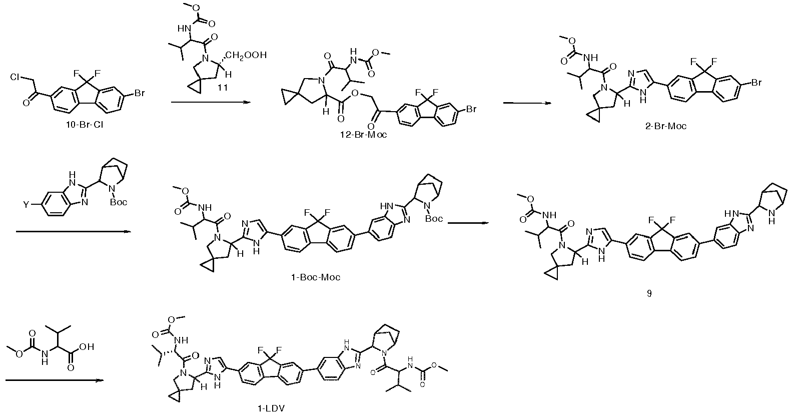

In this method to the compound of formula 11 is first introduced Moc-val group without Boc protection, which can significantly improve the synthesis efficiency and reduce three waste emissions.

### Route 2

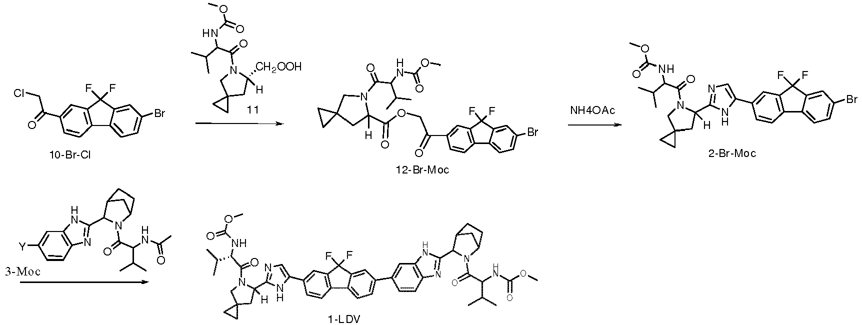

In this method, to a compound of formula 11 and a compound of formula 3 are first introduced Moc-Val, of which the protection and deprotection reaction are abolished, thus significantly reducing the synthesis steps, improve the synthesis efficiency, significantly reduce the production cycle, significantly reduce three waste emissions and greatly reduce the cost of raw materials, thereby this method has great industrial significance.

### Route 3

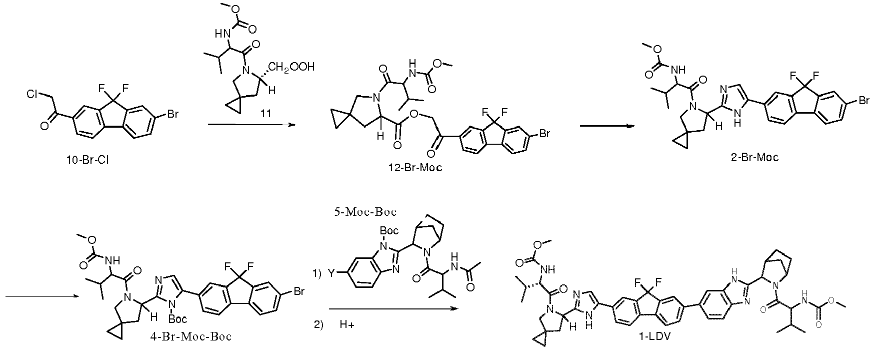

In this method the protection group is introduced in the compound 4-Br-Moc-Boc and compound 5-Moc-Boc, which can reduce the influence of electron rich N atoms on the catalyst quantity, greatly reduce the amount of catalyst, promote the reaction, and increase the utilization of raw materials. Since the catalysts and materials are expensive, this route can significantly reduce the cost of raw materials. At the same time, the route also reduces the content of defluorinated impurity in the product.

| Preparatio n method | Advantage |
|---|---|
| 1a | The content of defluorinated impurity can be effectively reduced, and the purity of the product can be greatly improved |
| 1b | The proportion of defluorinated impurity can be effectively reduced, and the purity of the product can be greatly improved; the dosage of the catalyst is greatly reduced, the cost of raw material is reduced, and the heavy metal is easy to be removed. |
| 2a | The content of defluorinated impurity can be effectively reduced, and the purity of the product can be greatly improved |
| 2b | The content of defluorinated impurity can be effectively reduced, and the purity of the product can be greatly improved |
| 2c | The content of defluorinated impurity can be effectively reduced, and the purity of the product can be greatly improved |
| 2d | The content of defluorinated impurity can be effectively reduced, and the purity of the product can be greatly improved |
| 3a | the synthesis steps are significantly reduced, and the synthetic routes are most efficient, which greatly improve the synthesis efficiency, significantly decrease the production cycle, significantly reduce waste emissions, substantially reduce raw material costs, thereby has great industrial significance. |
| 3b | the amount of catalyst is further reduced comparing to 3a, the cost of raw material is reduced, and the heavy metal is removed easily. |
| 4a | Boc protection is abolished, reaction steps are reduced, the synthesis efficiency is significantly improved, waste emissions and the cost of raw material are reduced. |
| 4b | the amount of catalyst is further reduced comparing to 4a, the cost of raw material is reduced, and the heavy metal is removed easily. |

The invention will now be further described with reference to specific embodiments. It should be understood that these examples are merely illustrative of the invention and are not intended to limit the scope of the invention. Any experimental method that are not specified with conditions in the following embodiments, usually is conducted in accordance with conventional conditions or under the conditions proposed by the manufacturer. Unless otherwise stated, percentages and fractions are calculated by weight.

### 1. imidazole group without protective group

### Example 1: synthesis of compound 12-Br-Cbz

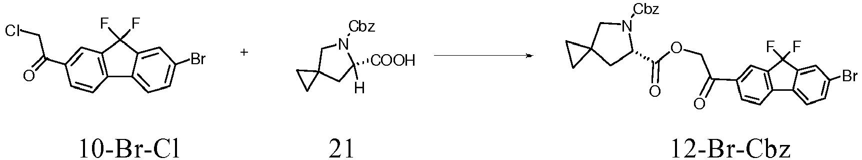

Compound 10-Br-Cl (2.03 g, 5.675 mmol), compound 21 (1.72 g, 6.243 mmol), DIPEA (0.81 g, 6.243 mmol) and acetonitrile (40 mL) were added to a three-necked bottle, then heated to 70 °C and stirred for 5 hours. After that, the mixture was cooled to room temperature. After the solvent was distilled off, ethyl acetate (100 mL) was added and the mixture was washed with dilute hydrochloric acid (0.01 M / L, 200 mL). The organic phase was dried over anhydrous sodium sulfate and the solvent was distilled off to obtain the product (3.384 g, yield 100%).

### Example 2: synthesis of compound 2-Br-Cbz

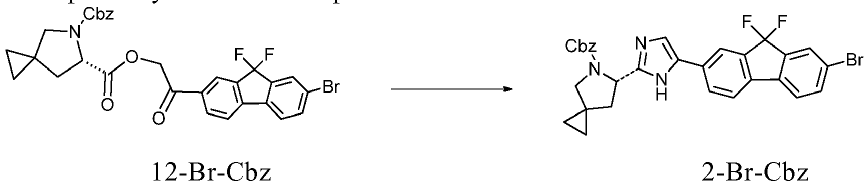

Compound 12-Br-Cbz (3.384g, 5.675mmol), ammonium acetate (2.187g, 28.375mmol), ethylene glycol monomethyl ether (4mL) and toluene (70mL) were added into a three-necked bottle, then heated to 90 °C and stirred for 5 hours. After that the mixture was cooled to room temperature, and then ethyl acetate (100mL) was added. The mixture was washed with brine (200 mL) twice. The organic phase was dried with anhydrous sodium sulfate and the solvent was distilled off to obtain the product (3.2g, yield 98%).

### Example 3: synthesis of compound 1-Cbz-Cbz

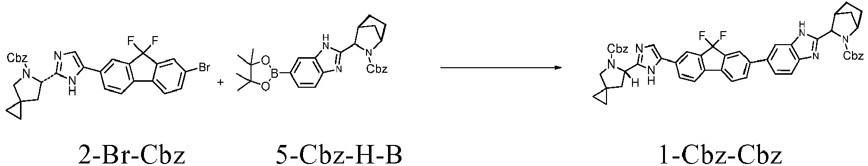

Compound 2-Br-Cbz (3.0 g, 5.2 mmol), compound 5-Cbz-H-B (2.71 g, 5.72 mmol), PdCl₂(dppf) (0.19 g, 0.26 mmol), potassium carbonate (2.156g, 15.6mmol),water(10 mL) and dioxane (50 mL) were added into a three-necked bottle. Under nitrogen the mixture was heated to 90 °C and stirred for 16 hours. After that the mixture was cooled to room temperature and ethyl acetate (100 mL) was added. The mixture was washed with brine (200 mL) twice.The organic phase was dried over anhydrous sodium sulfate and the solvent was distilled off to give the product (3.945 g, yield 90%), in which the content of the product is 98.4% and the content of defluorinated impurity is 0.23% (220 nm).

### Example 4: synthesis of compound 6

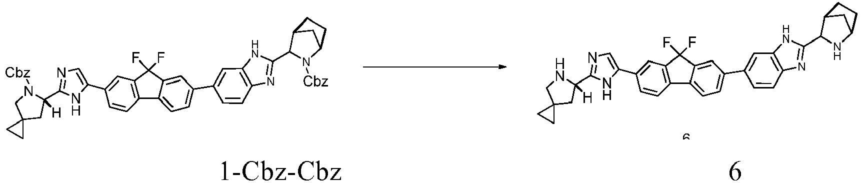

Compound 1-Cbz-Cbz (3.8 g, 4.5 mmol), Pd / C (0.38 g, 10%) and methanol (50 mL) were added to an autoclave, which was pressurized to 150 psi with hydrogen and stirred at room temperature for 16 hours. The mixture was then filtered and the filtrate was concentrated to obtain the product (2.586 g, yield 100%), in which the content of compound 6 is 98.3% and the content of defluorinated impurity is 0.27% (220 nm).

### Example 5: synthesis of compound 12-Br

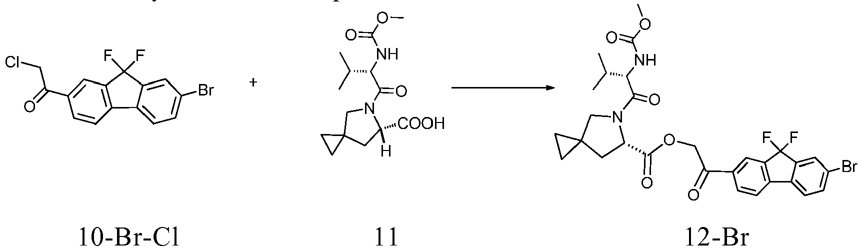

Compound 10-Br-Cl (2.03 g, 5.675 mmol), compound 11 (1.86 g, 6.243 mmol), DIPEA (0.81 g, 6.243 mmol) and acetonitrile (40 mL) were added to a three-necked bottle, then heated to 70 °C and stirred for 5 hours. After that, the mixture was cooled to room temperature. After the solvent was distilled off, ethyl acetate (100 mL) was added and the mixture was washed with dilute hydrochloric acid (0.01 M / L, 200 mL). The organic phase was dried over anhydrous sodium sulfate and the solvent was distilled off to obtain the product (3.5 g, yield 100%).

### Example 6: synthesis of compound 13-Br-Moc

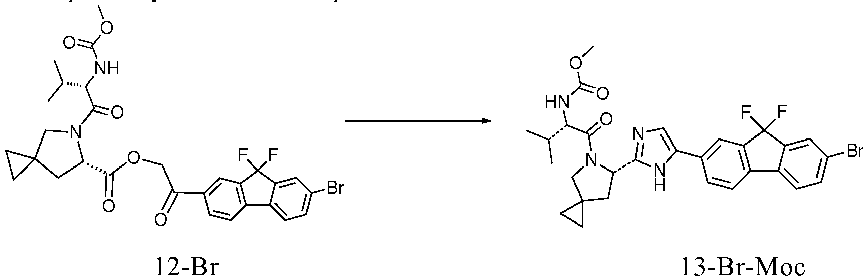

Compound 12-Br (3.5g, 5.675mmol), ammonium acetate (2.187g, 28.375mmol), ethylene glycol monomethyl ether (4mL) and toluene (70mL) were added into a three-necked bottle, then heated to 90 °C and stirred for 5 hours. After that the mixture was cooled to room temperature, and then ethyl acetate (100mL) was added. The mixture was washed with brine (200 mL) twice. The organic phase was dried with anhydrous sodium sulfate and the solvent was distilled off to obtain the product (3.4g, yield 100%).

### Example 7: synthesis of compound 1-LDV

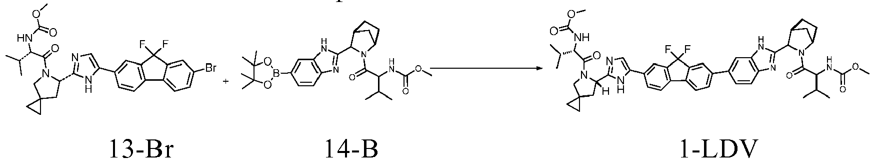

Compound 13-Br (3.4 g, 5.675 mmol), compound 14-B (3.1 g, 6.243 mmol), PdCl₂(dppf) (0.141 g, 0.2838 mmol), potassium carbonate (2.353 g, 17.025 mmol), water (10 mL) and tert-amyl alcohol (50 mL) were added into a three-necked bottle. Under nitrogen the mixture was heated to 90 °C and stirred for 16 hours. After that the mixture was cooled to room temperature and ethyl acetate (100 mL) was added. The mixture was washed with brine (200 mL) twice. The organic phase was dried over anhydrous sodium sulfate and the solvent was distilled off to give the product (4.54 g, yield 90%), in which the content of compound 1-LDV is 99.3% and the content of defluorinated impurity is 0.13% (220nm).

### Example 8: synthesis of compound 18

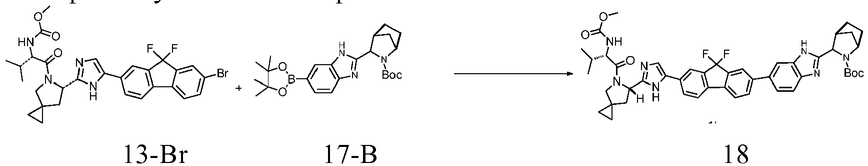

Compound 13-Br (3.4 g, 5.675 mmol), compound 17-B (3.1 g, 6.243 mmol), Pd(PPh₃)₄ (0.328 g, 0.2838 mmol), potassium carbonate (2.353 g, 17.025 mmol), water (10 mL) and tert-amyl alcohol (50 mL) were added into a three-necked bottle. Under nitrogen the mixture was heated to 90 °C and stirred for 16 hours. After that the mixture was cooled to room temperature and ethyl acetate (100 mL) was added. The mixture was washed with brine (200 mL) twice. The organic phase was dried over anhydrous sodium sulfate and the solvent was distilled off to give the product (4.34 g, yield 92%), in which the content of compound 18 is 97% and the content of defluorinated impurity is 0.26% (220nm).

### Example 9: synthesis of compound 9

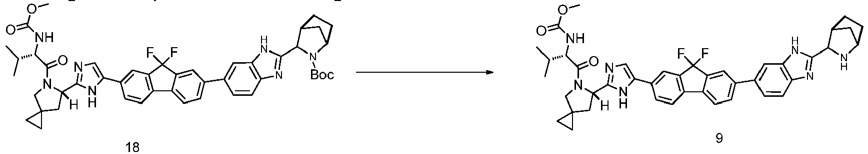

To a three-necked flask was added compound 18 (4.16 g, 5 mmol) and DCM (40 mL), and a solution of HCl in dioxane (4 M / L, 40 mL) was added dropwise with stirring at 10 °C. After that the mixture was stirred for 5 hours. Then the solvent was distilled off to give the product of Compound 9 hydrochloride (4.2 g, yield 100%), in which the content of compound 9 is 98.5% and the content of defluorinated impurity is 0.12% (220 nm).

### Example 10: synthesis of compound 1-LDV

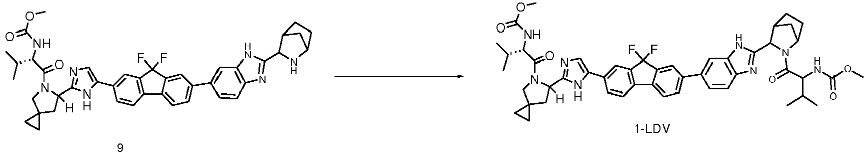

Compound 9 hydrochloride (4.2 g, 5 mmol), (S)-2-methoxycarbonylamino-3-methyl-butyric acid (0.963 g, 5.5 mmol), EDCI (1.437 g, 7.5mmol), HOBt (1.013g, 7.5mmol), triethylamine (2.53g, 25mmol) and DMF (100 mL) were added into a three-necked bottle. The mixture was stirred at 20 °C for 16 hours. After that ethyl acetate (100mL) was added and the mixture was washed with brine (200 mL) twice. The organic phase was dried over anhydrous sodium sulfate and the solvent was distilled off to give the product (4.0 g, yield 90%).in which the content of compound 1-LDV is 99% and the content of defluorinated impurity is 0.15% (220 nm).

### Example 11: synthesis of compound 1-Cbz-Moc

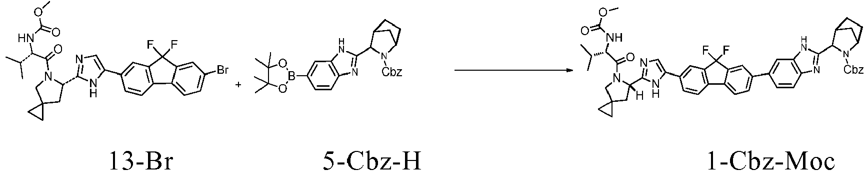

Compound 13-Br (3.4 g, 5.675 mmol), compound 5-Cbz-H (2.955 g, 6.243 mmol), PdCl₂(dppf) (0.141 g, 0.2838 mmol), potassium carbonate (2.353 g, 17.025 mmol), water (10 mL) and tert-amyl alcohol (50 mL) were added into a three-necked bottle. Under nitrogen the mixture was heated to 90 °C and stirred for 16 hours. After that the mixture was cooled to room temperature and ethyl acetate (100 mL) was added. The mixture was washed with brine (200 mL) twice. The organic phase was dried over anhydrous sodium sulfate and the solvent was distilled off to give the product (4.275 g, yield 87%), in which the content of compound 1-Cbz-Moc is 98% and the content of defluorinated impurity is 0.2% (220 nm).

### Example 12: synthesis of compound 9

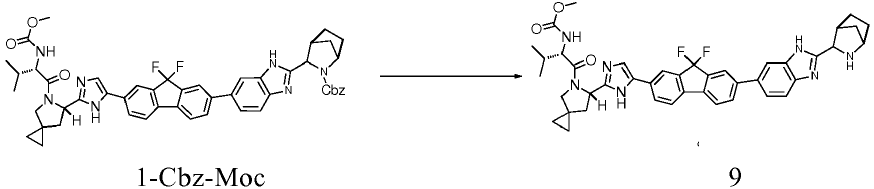

Compound 1 (4.91 g, 5.675 mmol), Pd / C (0.49 g, 10%) and methanol (50 mL) were added to an autoclave, which was pressurized to 150 psi with hydrogen and stirred at room temperature for 16 hours. The mixture was then filtered and the filtrate was concentrated to obtain the product (3.945 g, yield 95%), in which the content of compound 9 is 99% and the content of defluorinated impurity is 0.14% (220 nm).

### Comparative example 11: (see JMC, 2014:2033)

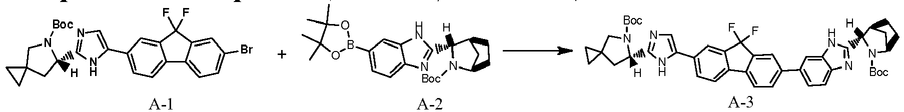

2.83 g of compound A-1, 2.75 g of compounds A-2, 78 mg of Pd (OAc)₂, 155 mg of triphenylphosphine, 56 mL of DME and 20 mL of sodium carbonate solution (1M). The reaction mixture was replaced by nitrogen for 5 times, then heated to 93 °C and reacted for 4 hours at that temperature. Then the mixture was cooled to room temperature and quenched by saturated sodium bicarbonate (100mL), then extracted with ethyl acetate (150 mL x 2). The organic phase was washed with brine (100mL x 2) twice and dried with anhydrous sodium sulfate, and the solvent was distilled off to give the product (4.0 g, yield 95%).

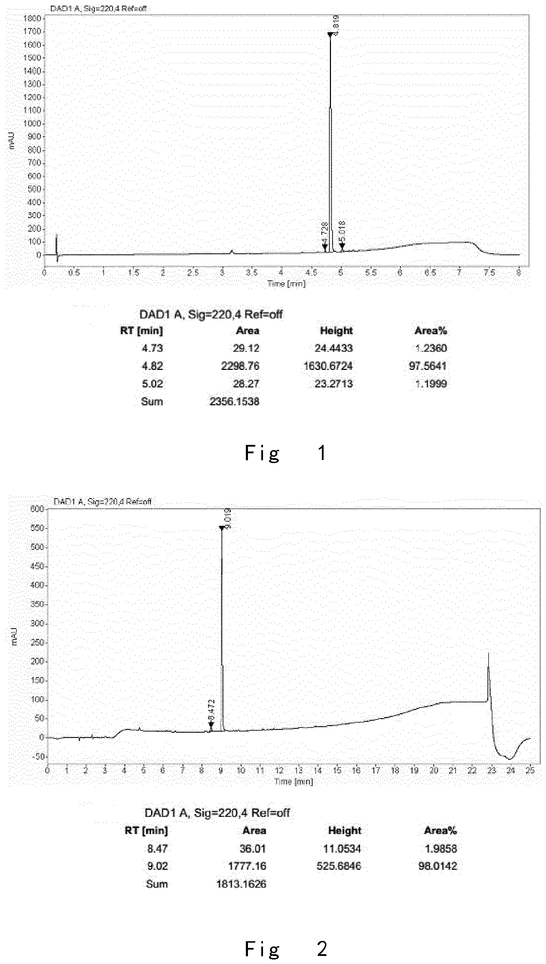

The content of defluorinated impurity in the product is about 1.2% (220nm), as shown in figure 1.

### Comparative example 12: (see US2013314740)

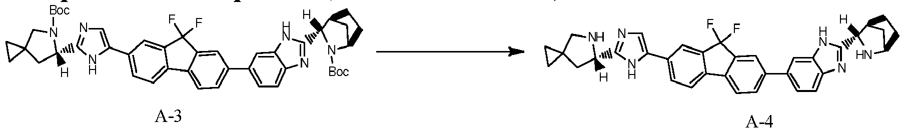

4.5 g of compound A-3 was dissolved in 15.8 mL of acetonitrile, to which 20 mL of 1.5N hydrochloric acid was added. The mixture was heated to 65 °C and stirred for 2 hours at that temperature. The reaction was monitored by HPLC till the reaction was finished. The mixture was cooled to 45 °C, then 103.5 mL of acetonitrile was added dropwise and the temperature of the reaction solution was controlled at 40 to 50 °C during the addition. The resulting turbidity was cooled to 20 °C and stirred for 2 hours at that temperature. The solid obtained by filtration was dried in vacuo to give 4.0 g of compound A-4.

The content of defluorinated impurity in the product is about 1.6- 2.0% (220nm). See figure 2.

### 2. imidazole group with protective group

### Example 13:

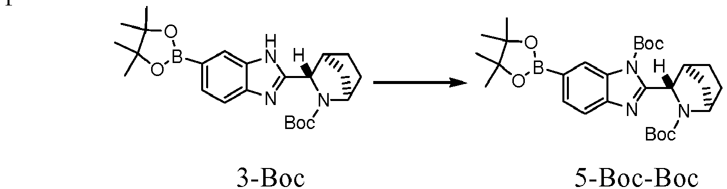

Into a 50 mL three-necked bottle, 0.46 g (1 mmol, 1 eq) of compound 3-Boc, a catalytic amount of N, N-dimethylaminopyridine (DMAP), and 10 mL of dichloromethane were added and stirred till dissolved. 0.26 g (1.2 mmol, 1.2 eq) of Boc anhydride (pre-dissolved in 5 mL of dichloromethane) was added dropwise at room temperature. After the addtion was finished, the mixture was stirred at room temperature for 15 hours. The reaction solution was washed with 5% brine (5 mL x 3), dried over anhydrous sodium sulfate and concentrated under reduced pressure to give 0.52 g of 5-Boc-Boc as a white solid (yield: 96.3%). ¹H NMR (400 MHz, CDCl₃) δ 7.57 (s, 1H), 6.47 (s, 1H), 4.80 (s, 1H), 3.21 (s, 1H), 1.86 (d, J = 11.3 Hz, 1H), 1.65-1.57 (m, 10H), 1.42 (s, 9H), 1.37-1.04 (m, 14H), 1.11-1.04 (m, 1H).

### Example 14

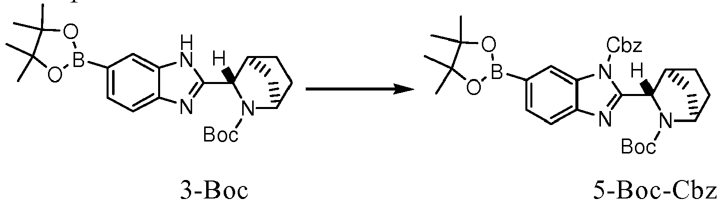

Into a 50 mL three-necked bottle, 0.46 g (1 mmol, 1 eq) of compound 3-Boc, a catalytic amount of N, N-dimethylaminopyridine (DMAP), and 10 mL of dichloromethane were added and stirred till dissolved. 0.19 g (1.5 mmol, 1.5 eq) of acetylchloride (pre-dissolved in 3 mL of dichloromethane) was added dropwise at room temperature. After the addtion was finished, the mixture was stirred at room temperature for 15 hours. The reaction solution was washed with 5% sodium carbonate solution (5 mL x 3) and 5% brine (5 mL x 3), dried over anhydrous sodium sulfate and concentrated under reduced pressure to give 0.55 g of 5-Boc-Cbz as a white solid (yield: 96.0%). ¹H NMR (400 MHz, CDCl₃) δ 7.57 (s, 1H), 7.32 (d, J = 4.0 Hz, 5H), 6.46 (s, 1H), 5.13 (s, 2H), 4.82 (s, 1H), 3.27 (s, 1H), 1.87 (d, J = 7.4 Hz, 1H), 1.61 (d, J = 12.0 Hz, 2H), 1.65-1.29 (m, 13H), 1.14 (s, 12H).

### Example 15

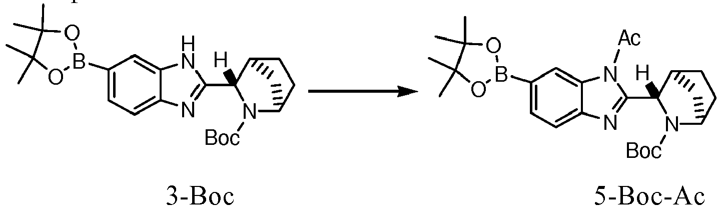

Into a 50 mL three-necked bottle, 0.46 g (1.0 mmol, 1 eq) of compound 3-Boc, a catalytic amount of N, N-dimethylaminopyridine (DMAP), and 10 mL of dichloromethane were added and stirred till dissolved. 0.12 g (1.5 mmol, 1.5 eq) of acetylchloride (pre-dissolved in 5 mL of dichloromethane) was added dropwise at room temperature. After the addtion was finished, the mixture was stirred at room temperature for 15 hours. The reaction solution was washed with 5% sodium carbonate solution (5 mL x 3) and 5% brine (5 mL x 3), dried over anhydrous sodium sulfate and concentrated under reduced pressure to give 0.45 g of 5-Boc-Ac as a white solid (yield: 93.7%). ¹H NMR (400 MHz, CDCl₃) δ 7.57 (s, 1H), 6.24 (s, 1H), 4.80 (s, 1H), 3.35 (s, 1H), 2.65 (s, 3H), 1.87 (d, J = 5.8 Hz, 1H), 1.61 (d, J = 12.0 Hz, 1H), 1.42 (s, 9H), 1.33 (d, J = 16.0 Hz, 1H), 1.14 (s, 12H).

### Example 16

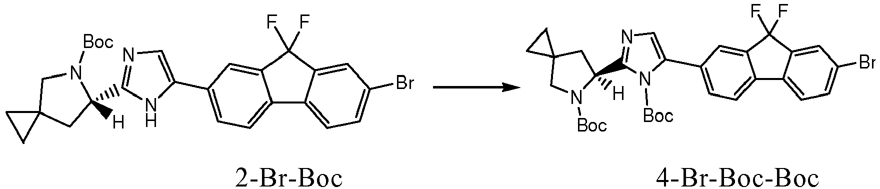

Into a 50 mL three-necked bottle, 0.54 g (1 mmol, 1 eq) of compound 2-Br-Boc, a catalytic amount of N, N-dimethylaminopyridine (DMAP), and 15 mL of dichloromethane were added and stirred till dissolved. 0.26 g (1.2 mmol, 1.2 eq) of Boc anhydride (pre-dissolved in 5 mL of dichloromethane) was added dropwise at room temperature. After the addtion was finished, the mixture was stirred at room temperature for 15 hours. The reaction solution was washed with 5% brine (5 mL x 3), dried over anhydrous sodium sulfate and concentrated under reduced pressure to give 0.62 g of 4-Br-Boc-Boc as a white solid (yield: 96.9%). ¹H NMR (400 MHz, CDCl₃) δ 8.09 (s, 1H), 7.79 (d, J = 4.0 Hz, 2H), 7.35 (s, 1H), 6.80 (s, 1H), 6.03 (s, 1H), 5.97 (s, 1H), 5.13 (s, 1H), 4.07 (d, J = 15.2 Hz, 2H), 2.43 (s, 2H), 1.63 (s, 9H), 1.42 (s, 9H), 0.71 (d, J = 15.2 Hz, 4H).

### Example 17

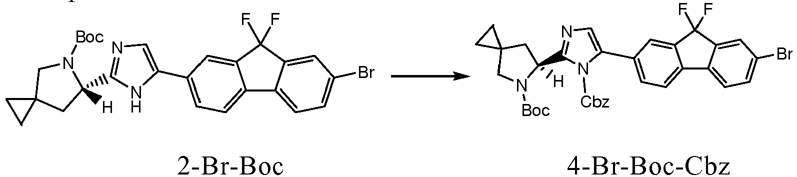

Into a 50 mL three-necked bottle, 0.54 g (1 mmol, 1 eq) of compound 2-Br-Boc, a catalytic amount of N, N-dimethylaminopyridine (DMAP), and 15 mL of dichloromethane were added and stirred till dissolved. 0.19 g (1.5 mmol, 1.5 eq) of benzyl chloroformate (pre-dissolved in 3 mL of dichloromethane) was added dropwise at room temperature. After the addtion was finished, the mixture was stirred at room temperature for 15 hours. The reaction solution was washed with 5% sodium carbonate solution (5 mL x 3) and 5% brine (5 mL x 3), dried over anhydrous sodium sulfate and concentrated under reduced pressure to give 0.65 g of 4-Br-Boc-Cbz as a white solid (yield: 96.3%). ¹H NMR (400 MHz, CDCl₃) δ 8.09 (s, 1H), 7.80 (dd, J = 8.5, 8.1 Hz, 6H), 7.63 (s, 1H), 7.33 (t, J = 6.0 Hz, 9H), 6.78 (s, 1H), 5.13 (s, 3H), 3.96 (d, J = 54.8 Hz, 3H), 2.28 (s, 3H), 1.42 (s, 12H), 0.72 (s, 2H), 0.65 (s, 2H).

### Example 18

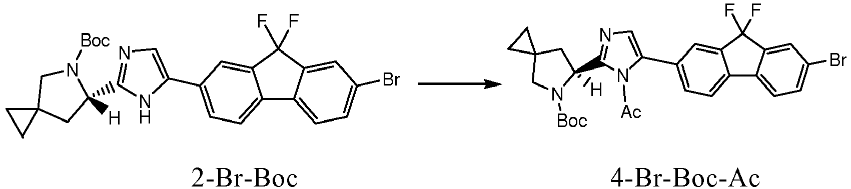

Into a 50 mL three-necked bottle, 0.54 g (1 mmol, 1 eq) of compound 2-Br-Boc, a catalytic amount of N, N-dimethylaminopyridine (DMAP), and 15 mL of dichloromethane were added and stirred till dissolved. 0.19 g (1.5 mmol, 1.5 eq) of acetylchloride (pre-dissolved in 3 mL of dichloromethane) was added dropwise at room temperature. After the addtion was finished, the mixture was stirred at room temperature for 15 hours. The reaction solution was washed with 5% sodium carbonate solution (5 mL x 3) and 5% brine (5 mL x 3), dried over anhydrous sodium sulfate and concentrated under reduced pressure to give 0.52 g of 4-Br-Boc-Ac as a white solid (yield: 89.7%). ¹H NMR (400 MHz, CDCl₃) δ 8.09 (s, 1H), 7.79 (d, J = 4.0 Hz, 2H), 7.66 (d, J = 9.4 Hz, 2H), 7.47 (d, J = 2.9 Hz, 2H), 6.37 (s, 1H), 3.92 (d, J = 52.8 Hz, 2H), 2.65 (s, 3H), 2.40 (s, 2H), 1.42 (s, 9H), 0.76 (s, 2H), 0.66 (s, 2H).

### Example 19

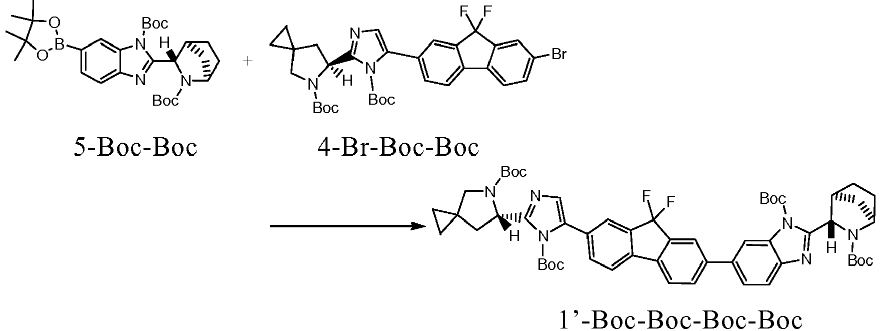

Into a 25 mL eggplant-type reaction flask, 300 mg (0.47 mmol, 1.0 eq) of compound 4-Br-Boc-Boc, 270 mg (0.49 mmol, 1.07 eq) of compound 5-Boc-Boc, 5 mL of amylene alcohol, 3 mg (1mol%) of PdCl₂(dppf), and 1 mL of 1 mol / L potassium phosphate solution were added. The reaction mixture was purged with nitrogen three times, then heated to 85 °C and reacted at this temperature. The reaction was monitored by HPLC till the reaction was complete when the compound 4-Br-Boc-Boc was consumed. The reaction solution was concentrated under reduced pressure till dry, to which 5 mL of ethyl acetate was added for refining. The mixture was then filtered through a silica gel-equipped sand core funnel to remove the black material to give a pale yellow solution which was concentrated under reduced pressure to give 420 mg of crude 1'-Boc-Boc-Boc-Boc. No further purification is required to proceed directly to the next step. ¹H NMR (400 MHz, CDCl₃) δ 8.90 (s, 1H), 8.19 (s, 1H), 8.11 (s, 1H), 8.06 (d, J = 20.2 Hz, 2H), 7.91 (s, 1H), 7.77 (d, J = 8.0 Hz, 3H), 7.35 (s, 1H), 6.90 (s, 1H), 6.28 (s, 1H), 4.85 (s, 1H), 4.10 (s, 1H), 4.04 (s, 1H), 3.47 (s, 1H), 2.45 (s, 2H), 1.99 (s, 1H), 1.88 (s, 1H), 1.65-1.57 (m, 19H), 1.42 (s, 18H), 1.36 (d, J = 9.0 Hz, 2H), 0.72 (d, J = 19.5 Hz, 4H).

### Example 20

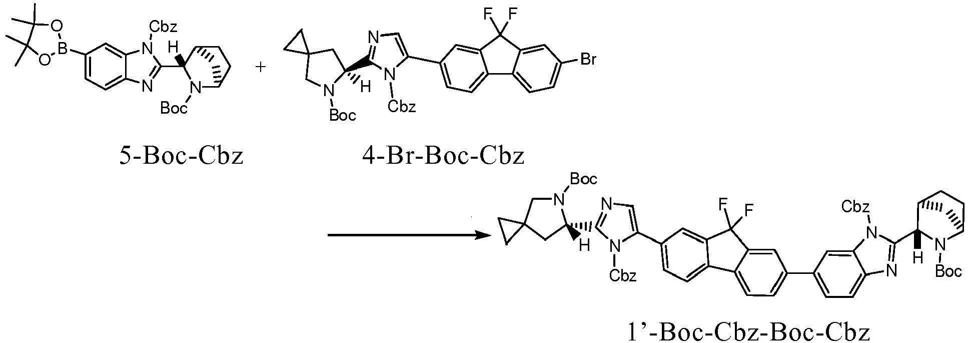

Into a 25 mL eggplant-type reaction flask, 220 mg (0.326 mmol, 1.0 eq) of compound 4-Br-Boc-Cbz, 200 mg (0.349 mmol, 1.08 eq) of compound 5-Boc-Cbz, 5 mL of amylene alcohol, 3.8 mg (1mol%) of Pd(PPh₃)₄, and 1 mL of 1M potassium phosphate solution were added. The reaction mixture was purged with nitrogen three times, then heated to 85 °C and reacted at this temperature. The reaction was monitored by HPLC till the reaction was complete when the compound 4-Br-Boc-Cbz was consumed. The reaction solution was concentrated under reduced pressure till dry, to which was added 5 mL of ethyl acetate for refining. The mixture was then filtered through a silica gel-equipped sand core funnel to remove the black material to give a pale yellow solution which was concentrated under reduced pressure to give 310 mg of crude 1'-Boc-Cbz-Boc-Cbz. No further purification is required to proceed directly to the next step. ¹H NMR (400 MHz, CDCl₃) δ 8.46 (s, 1H), 8.19 (s, 1H), 8.09 (s, 2H), 7.95 (d, J = 19.1 Hz, 2H), 7.77 (d, J = 8.0 Hz, 3H), 7.37-7.25 (m, 11H), 6.74 (s, 1H), 6.59 (s, 1H), 5.13 (s, 4H), 4.85 (s, 1H), 4.01 (d, J = 39.4 Hz, 2H), 3.27 (s, 1H), 2.17 (s, 2H), 1.88 (d, J = 1.8 Hz, 1H), 1.74-1.65 (m, 1H), 1.61 (d, J = 12.0 Hz, 2H), 1.74-1.30 (m, 22H), 0.69 (s, 2H), 0.62 (s, 2H).

### Example 21

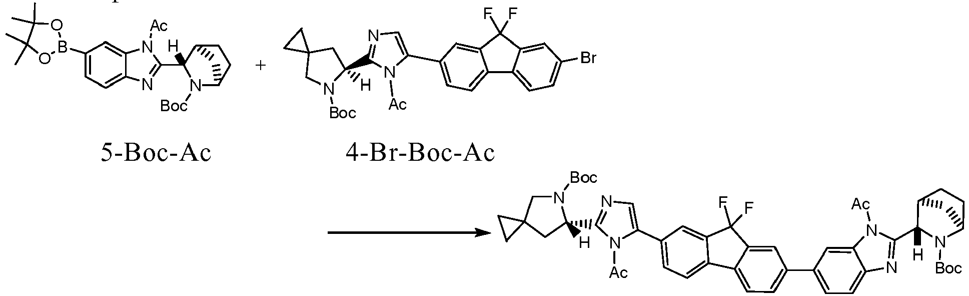

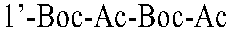

Into a 25 mL eggplant-type reaction flask, 110 mg (0.19 mmol, 1.0 eq) of compound 4-Br-Boc-Ac, 100 mg (0.21 mmol, 1.1 eq) of compound 5-Boc-Ac, 5 mL of amylene alcohol, 2.2 mg (1mol%) of PdCl₂(dppf), and 1 mL of 1 M potassium phosphate solution were added. The reaction mixture was purged with nitrogen three times, then heated to 85 °C and reacted at this temperature. The reaction was monitored by HPLC till the reaction was complete when the compound 4-Br-Boc-Ac was consumed. The reaction solution was concentrated under reduced pressure till dry, to which was added 5 mL of ethyl acetate for refining. The mixture was then filtered through a silica gel-equipped sand core funnel to remove the black material to give a pale yellow solution which was concentrated under reduced pressure to give 160 mg of crude 1'-Boc-Ac-Boc-Ac. No further purification is required to proceed directly to the next step. ¹H NMR (400 MHz, CDCl₃) δ 8.28 (s, 1H), 8.20 (d, J = 1.6 Hz, 1H), 8.14 (d, J = 40.0 Hz, 3H), 7.98-7.95 (m, 2H), 7.77 (d, J = 8.0 Hz, 3H), 7.47 (s, 1H), 6.51 (s, 1H), 5.30 (s, 1H), 4.88 (s, 1H), 3.90 (s, 2H), 3.85 (s, 1H), 3.39 (s, 1H), 2.65 (s, 6H), 2.34 (s, 2H), 2.24 (s, 1H), 1.88 (s, 1H), 1.61 (d, J = 12.0 Hz, 2H), 1.42 (s, 18H), 1.35 (s, 1H), 1.28 (s, 1H), 0.72 (s, 2H), 0.63 (s, 2H).

### Example 22

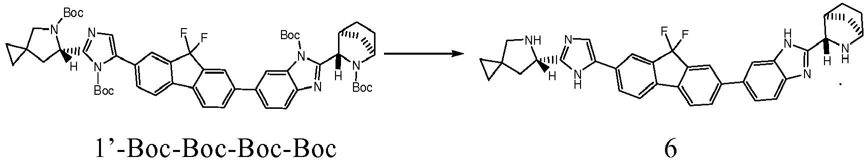

In a 25 mL one-necked eggplant-type reaction flask, 420 mg of compound 1'-Boc-Boc-Boc-Boc, 4.2 mL of acetonitrile, 3 mL of 1.5 M hydrochloric acid were added and reacted at 55 °C overnight. The reaction was monitored by HPLC till the starting material was completely consumed. 8.4 mL of acetonitrile was slowly added dropwise keeping the temperature at 40-50 °C. Then the mixture was cooled to room temperature and reacted for 2 hours. A white solid was obtained by filtration, washed with 1 mL of acetonitrile and dried to give 247 mg of compound 6 hydrochloride (yield 80%).

### Example 23

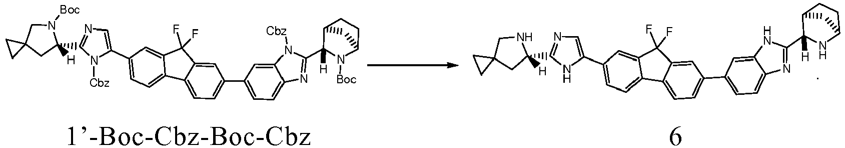

In a 25 mL one-necked eggplant-type reaction flask, 310 mg of compound 1'-Boc-Cbz-Boc-Cbz, 3.1 mL of acetonitrile, and 2.7 mL of 40% HBr-acetic acid solution were added and reacted at room temperature overnight. The reaction was monitored by HPLC till the starting material was completely consumed. 6.2 mL of acetonitrile was slowly added dropwise keeping the temperature at room temperature. After the addotion the mixture was stirred for another 2 hours. A white solid was obtained by filtration, washed with 1 mL of acetonitrile and dried to give 200 mg of compound 6 hydrobromide (yield 75.5%).

### Example 24

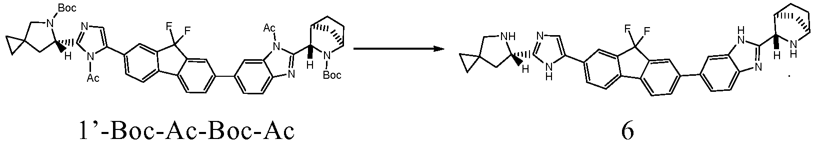

In a 25 mL one-necked eggplant-type reaction flask, 160 mg of compound 1'-Boc-Ac-Boc-Ac, 1.6 mL of acetonitrile and 1 mL of 6 M hydrochloric acid were added and reacted at 55 °C overnight. The reaction was monitored by HPLC till the starting material was completely consumed. 3.2 mL of acetonitrile was slowly added dropwise keeping the temperature at 40-50 °C. Then the mixture was cooled to room temperature and reacted for 2 hours. A white solid was obtained by filtration, washed with 1 mL of acetonitrile and dried to give 105 mg of compound 6 hydrochloride (yield 78.5%).

### Example 25: synthesis of compound 15-Br-Boc

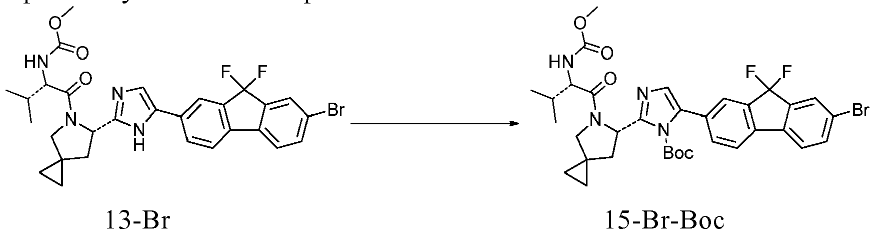

In a three-necked flask, compound 13-Br (3.4g, 5.675mmol), DMAP (0.139 g, 1.135 mmol), triethylamine (1.148 g, 11.35 mmol) and DCM (50 mL) were added, to which (Boc)₂O (2.477g, 11.35mmol) was added with stirring. After the addition, the mixture was stirred for 16 hours. Then DCM (100 mL) was added and the mixture was washed with brine (200 mL) twice. The organic phase was dried over anhydrous sodium sulfate and the solvent was distilled off to obtain the product (3.77 g, yield 95%).

### Example 26: synthesis of compound 16-B-Boc

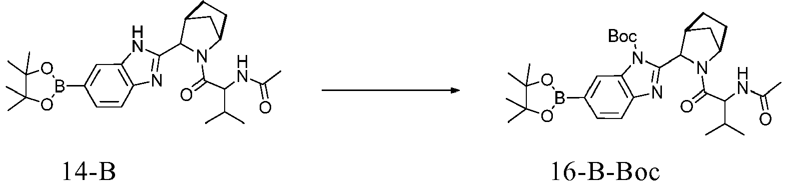

In a three-necked flask, compound 14-B (2.726g, 5.675mmol), DMAP (0.139 g, 1.135 mmol), triethylamine (1.148 g, 11.35 mmol) and DCM (50 mL) were added, to which (Boc)₂O (2.477g, 11.35mmol) was added with stirring. After the addition, the mixture was stirred for 16 hours. Then DCM (100 mL) was added and the mixture was washed with brine (200 mL) twice. The organic phase was dried over anhydrous sodium sulfate and the solvent was distilled off to obtain the product (3.13 g, yield 95%).

### Example 27: synthesis of compound 1'-LDV-Boc-Boc

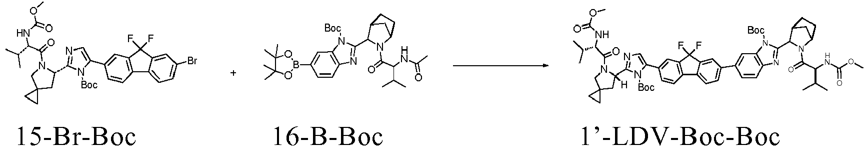

Compound 15-Br-Boc (3.5g, 5mmol), compound 16-B-Boc (3.19g, 5.5mmol), PdCl₂(dppf) (0.037 g, 0.05 mmol), potassium carbonate (2.073 g, 15 mmol), water (10 mL) and tert-amyl alcohol (50 mL) were added into a three-necked bottle. Under nitrogen the mixture was heated to 90 °C and stirred for 16 hours. After that the mixture was cooled to room temperature and ethyl acetate (100 mL) was added. The mixture was washed with brine (200 mL) twice. The organic phase was dried over anhydrous sodium sulfate and the solvent was distilled off to give the product (4.9 g, yield 90%).

### Example 28: synthesis of compound 1-LDV

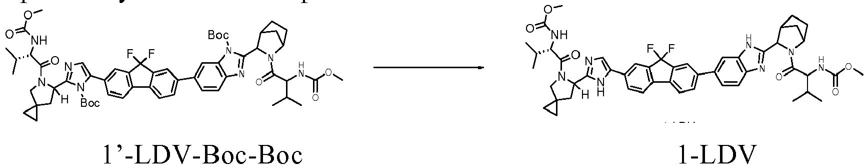

To a three-necked flask compound 1'-LDV-Boc-Boc (5.45g, 5mmol) and DCM (40 mL) were added, to which a solution of HCl in dioxane (4 M / L, 40 mL) was added dropwise with stirring at 10 °C. After the addition the mixture was stirred for 5 hours. Then the solvent was distilled off to give the product (4.44g, yield 100%).

### Example 29: synthesis of compound 15-Br-Bz

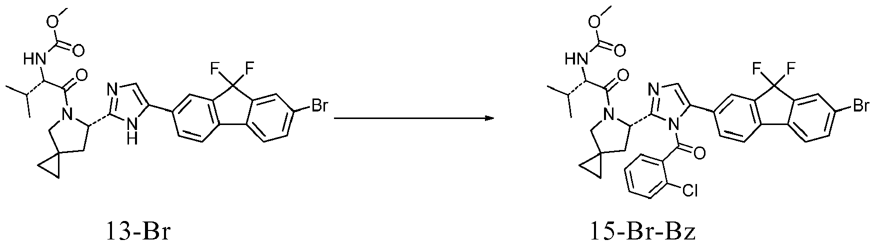

In a three-necked flask, compound 13-Br (3.4g, 5.675mmol), DMAP (0.139 g, 1.135 mmol), triethylamine (1.148 g, 11.35 mmol) and DCM (50 mL) were added, to which o-chlorobenzoyl chloride (1.99g, 11.35mmol) was added with stirring. After the addition, the mixture was stirred for 16 hours. Then DCM (100 mL) was added and the mixture was washed with brine (200 mL) twice. The organic phase was dried over anhydrous sodium sulfate and the solvent was distilled off to obtain the product (4.1 g, yield 98%).

### Example 30: synthesis of compound 16-B-Bz

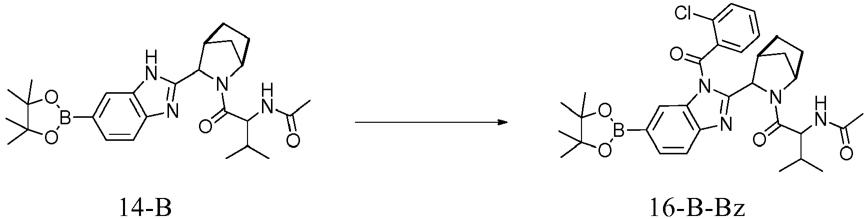

In a three-necked flask, compound 14-B (2.726g, 5.675mmol), DMAP (0.139 g, 1.135 mmol), triethylamine (1.148 g, 11.35 mmol) and DCM (50 mL) were added, to which o-chlorobenzoyl chloride (1.99g, 11.35mmol) was added with stirring. After the addition, the mixture was stirred for 16 hours. Then DCM (100 mL) was added and the mixture was washed with brine (200 mL) twice. The organic phase was dried over anhydrous sodium sulfate and the solvent was distilled off to obtain the product (3.34 g, yield 95%).

### Example 31: synthesis of compound 1'-LDV-Bz-Bz

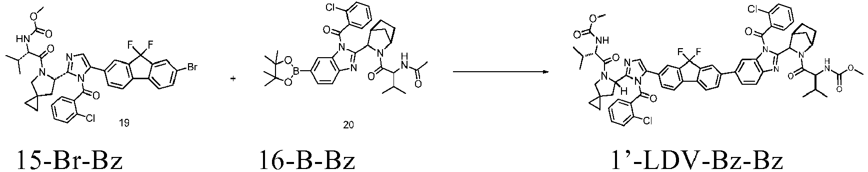

Compound 15-Br-Bz (3.69g, 5mmol), compound 16-B-Bz (3.4g, 5.5mmol), PdCl₂(dppf) (0.037 g, 0.05 mmol), potassium carbonate (2.073 g, 15 mmol), water (10 mL) and tert-amyl alcohol (50 mL) were added into a three-necked bottle. Under nitrogen the mixture was heated to 90 °C and stirred for 16 hours. After that the mixture was cooled to room temperature and ethyl acetate (100 mL) was added. The mixture was washed with brine (200 mL) twice. The organic phase was dried over anhydrous sodium sulfate and the solvent was distilled off to give the product (5.25 g, yield 90%).

### Example 32: synthesis of compound 1-LDV

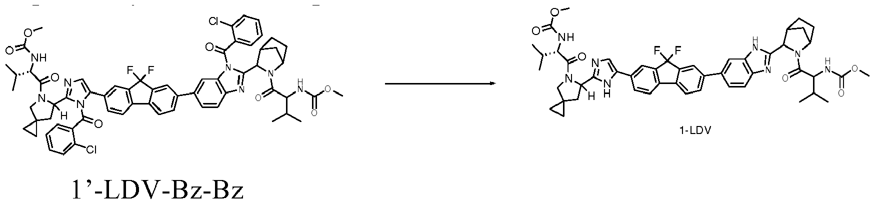

Compound 1'-LDV-Bz-Bz (5.25 g, 4.5 mmol), potassium phosphate aqueous solution (1M / L, 50 mL) and tert-amyl alcohol (50 mL) were added to a three-necked flask, and the temperature was raised to 90 °C. The mixture was stirred for 5 hours, then cooled to room temperature and extracted with ethyl acetate (100 mL). The organic phase was dried over anhydrous sodium sulfate and concentrated to give the product (4 g, yield 100%).

All documents mentioned in the present invention are incorporated herein by reference, as if each document were individually recited for reference. It should be understood that those skilled in the art will be able to make various changes or modifications to the present invention after reading the teachings of the present invention, which also fall within the scope of the claims appended hereto.

## Claims

1. A method of preparation for a compound of formula 1, wherein, 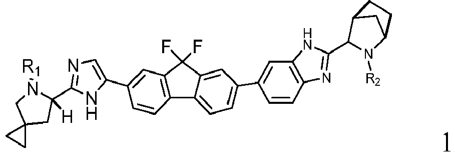
(1a) the method comprises steps of:
subjecting a compound of formula 2-1 with a compound of formula 3-1 to coupling reaction to give a compound of formula 1; 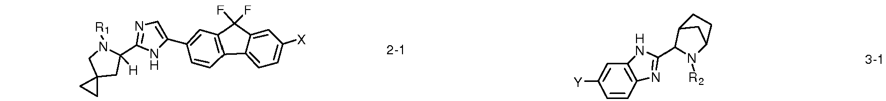 wherein,
R₁ and R₂ are defined as follows:
when R₁ is benzyloxycarbonyl (Cbz), R₂ is benzyloxycarbonyl (Cbz), t-butoxycarbonyl (Boc) or (S)-2-methoxycarbonylamino-3-methyl-butyryl (Moc-Val); or
when R₁ is t-butoxycarbonyl (Boc), R₂ is benzyloxycarbonyl (Cbz) or (S)-2-methoxycarbonylamino-3-methyl-butyryl (Moc-Val); or
when R₁ is (S)-2-methoxycarbonylamino-3-methyl-butyryl (Moc-Val), R₂ is benzyloxycarbonyl (Cbz) or (S)-2-methoxycarbonylamino-3-methyl-butyryl (Moc-Val);
X and Y are defined as follows:
when X is halogen (Cl, Br or I), Y is -B(OR₃)(OR₄) or -BF₃K; or
when X is -B(OR₃)(OR₄) or -BF₃K, Y is halogen (Cl, Br or I), wherein R₃ and R₄ are independently H or C 1-C6 linear or branched alkyl, or two of them together form C2-C8 linear or branched alkylidene;
or
(1b) the method comprises steps of:
(i) subjecting a compound of formula 2-2 to acylation reaction to give a compound of formula 4; 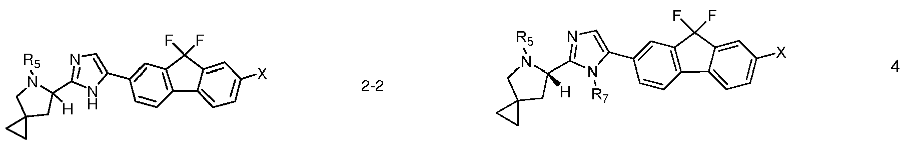 and subjecting a compound of formula 3-2 to acylation reaction to obtain a compound of formula 5; 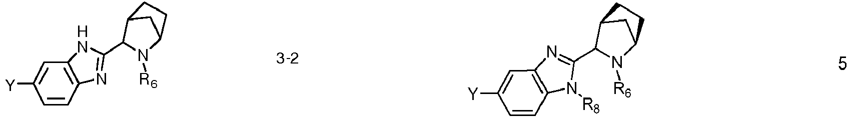
(ii) subjecting the compound of formula 4 with the compound of formula 5 to coupling reaction to obtain a mixture of a compound of formula 1' and a compound of formula 1; 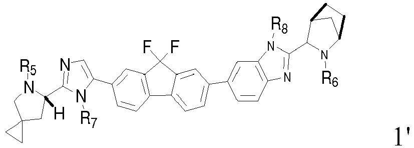
(iii) subjecting the mixture of a compound of formula 1' and a compound of formula 1 to hydrogenation reduction or hydrolysis reaction to obtain a compound of formula 1;
in each of the above scheme,
X and Y are defined as follows:
when X is halogen (Cl, Br or I), Y is -B(OR₃)(OR₄) or -BF₃K; or
when X is -B(OR₃)(OR₄) or -BF₃K, Y is halogen (Cl, Br or I), wherein R₃ and R₄ are independently H or C 1-C6 linear or branched alkyl, or two of them together form C2-C8 linear or branched alkylidene;
R₅ and R₆ are independently benzyloxycarbonyl (Cbz), t-butoxycarbonyl (Boc) or (S)-2-methoxycarbonylamino-3-methyl-butyryl (Moc-Val);
R₇ and R₈ are independently C1-C5 acyl, Cbz, Boc, or 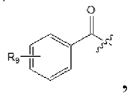 wherein, R₉ is a substituent at any position of benzene ring (ortho-, meta- or para-positionion), and the substituent is C1-C4 alkyl or halogen.

2. The method of claim 1, wherein, the method has one or more characteristics selected from the following group:
the coupling reaction is carried out in an inert solvent (such as THF, 2-methyl THF, dioxane, ether (C₁-C₄OC₁-C₄), C₁-C₅ alcohol or ester (C₁-C₄COOC₁-C₄));
the coupling reaction is carried out in the presence of Pd(0) or Pd(II) catalysts such as Pd(PPh₃)₄, PdCl₂(PPh₃)₂, Pd(OAc)₂, PdCl₂(P(t-Bu)₂Ph)₂, Pd(dppf)₂Cl₂ or its methylene dichloride complex;
the hydrogenation reaction is carried out in the presence of Pt, Pd or Ni catalyst;
the hydrolysis reaction is carried out in the presence of proton acid and / or Lewis acid.

3. A method of preparation for a compound of formula 1-LDV, wherein, 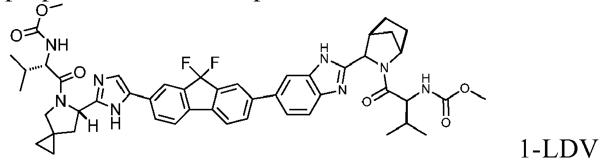
(3a) the method comprises steps of:
(i) subjecting a compound of formula 1 to hydrogenation reduction or hydrolysis reaction to obtain a compound of formula 6; 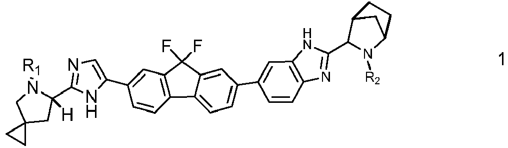 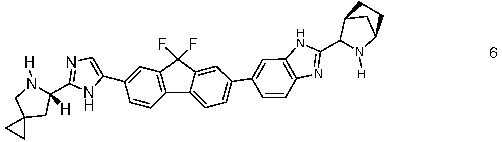 wherein, both of R₁ and R₂ are Cbz;
(ii) reacting the compound of formula 6 with (S)-2-methoxycarbonylamino-3-methyl-butyric acid to produce a compound of formula 1-LDV;
or
(3b) the method comprises steps of:
(i) subjecting a compound of formula 1 to hydrogenation reduction or hydrolysis reaction to obtain a compound of formula 7; 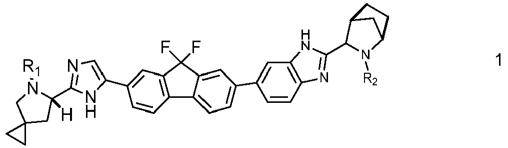 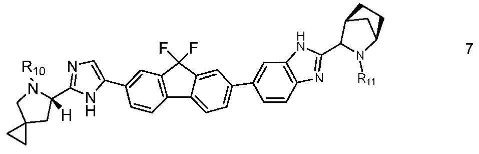 wherein,
when in compound 1 R₁ is Cbz and R₂ is Boc, in compound 7 R₁₀ is H and R₁₁ is Boc or H; or
when in compound 1 R₁ is Boc and R₂ is Cbz, in compound 7 R₁₀ is Boc or H and R₁₁ is H;
(ii) subjecting the compound of formula 7 to hydrolysis reaction to give a compound of formula 6; 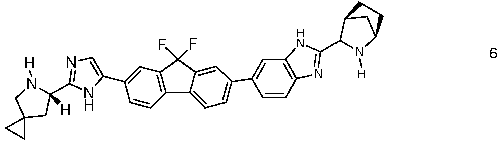
(iii) reacting the compound of formula 6 with (S)-2-methoxycarbonylamino-3-methyl-butyric acid to produce a compound of formula 1-LDV;
or
(3c) the method comprises steps of:
(i) subjecting a compound of formula 1 to hydrogenation reduction or hydrolysis to obtain a compound of formula 8; 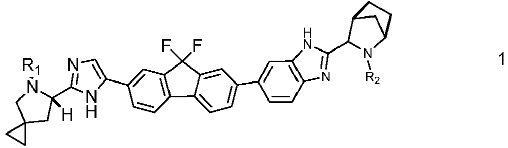 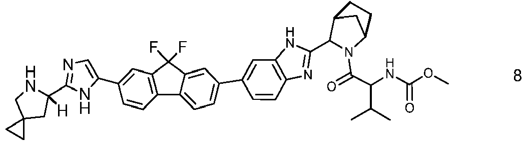 wherein, R₁ is Cbz or Boc; R₂ is Moc-Val;
(ii) reacting the compound of formula 8 with (S)-2-methoxycarbonylamino-3-methyl-butyric acid to produce a compound of formula 1-LDV;
or
(3d) the method comprises steps of:
(i) subjecting a compound of formula 1 to hydrogenation reduction or hydrolysis to obtain a compound of formula 9; 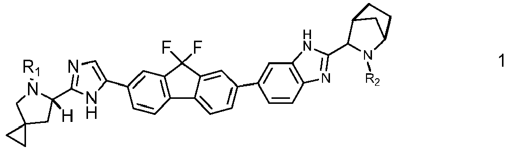 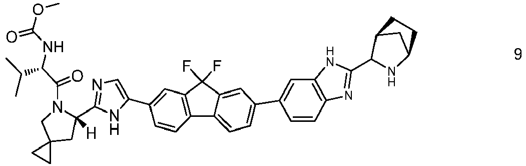 wherein, R₁ is Moc-Val; R₂ is Cbz.
(ii) reacting the compound of formula 9 with (S)-2-methoxycarbonylamino-3-methyl-butyric acid to produce a compound of formula 1-LDV;

4. The method of claim 3, wherein, the method has one or more characteristics selected from the group consisting of:
the hydrogenation reaction is carried out in the presence of Pt, Pd or Ni catalyst;
the hydrolysis reaction is carried out in the presence of proton acid and / or Lewis acid.

5. A method of preparation for a compound of formula 1-LDV, wherein, 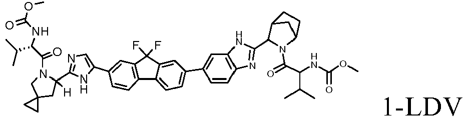
(5a) the method comprises steps of:
(i) reacting a compound of formula 10 with a compound of formula 11 to obtain a compound of formula 12; 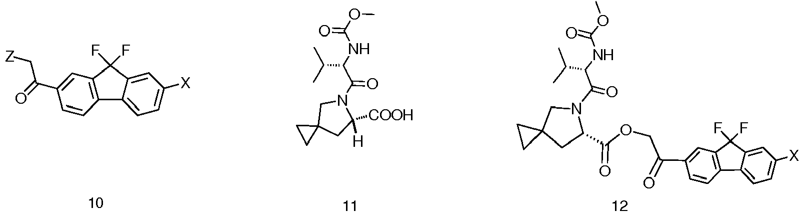
(ii) subjecting the compound of formula 12 with an ammonium salt to cyclization reaction to give a compound of formula 13; 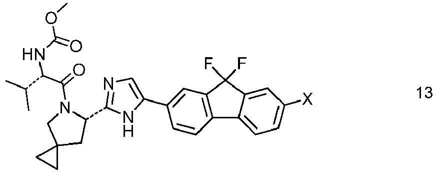
(iii) subjecting the compound of formula 13 with a compound of formula 14 to coupling reaction to give a compound of formula 1-LDV; 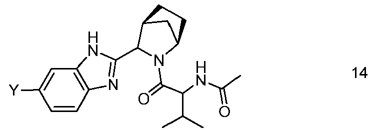 wherein,
when X is halogen (Cl, Br or I) then Y is -B(OR₃)(OR₄) or -BF₃K; or when X is -B(OR₃)(OR₄) or -BF₃K, then Y is halogen (Cl, Br or I), wherein R₃ and R₄ are independently H or C1-C6 linear or branched alkyl, or two of them together form C2-C8 linear or branched alkylidene;
Z is halogen (chlorine, bromine or iodine);
or
(5b) the method comprises steps of:
(i) reacting a compound of formula 10 with a compound of formula 11 to obtain a compound of formula 12; 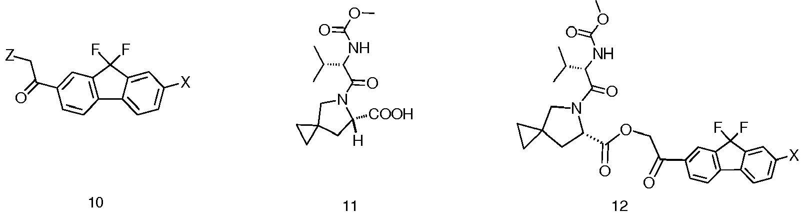
(ii) subjecting the compound of formula 12 with an ammonium salt to cyclization reaction to give a compound of formula 13; 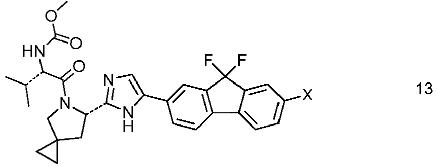
(iii) subjecting the compound of formula 13 to acylation reaction to produce a compound of formula 15; 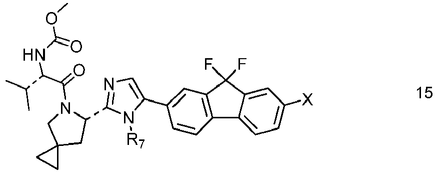 and subjecting a compound of formula 14 to acylation reaction to obtain a compound of formula 16; 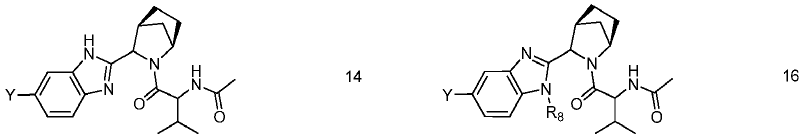
(iv) subjecting the compound of formula 15 with the compound of formula 16 to coupling reaction to obtain a mixture of a compound of formula 1'-LDV and a compound of formula 1-LDV; 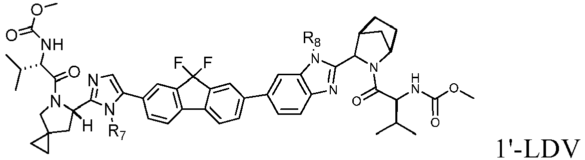
(v) subjecting the mixture of a compound of formula 1'-LDV and a compound of formula 1-LDV to hydrogenation reduction or hydrolysis to obtain a compound of formula 1-LDV;
wherein,
when X is halogen (Cl, Br or I), then Y is -B(OR₃)(OR₄) or -BF₃K; or when X is -B(OR₃)(OR₄) or -BF₃K, then Y is halogen (Cl, Br or I), wherein R₃ and R₄ are independently H or C1-C6 linear or branched alkyl, or two of them together form C2-C8 linear or branched alkylidene;
R₇ and R₈ are independently C1-C5 acyl, Cbz, Boc, or 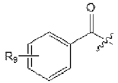 wherein, R₉ is a substituent at any position of benzene ring (ortho-, meta- or para-positionion), and the substituent is C1-C4 alkyl or halogen.

6. The method of claim 5, wherein, the method has one or more characteristics selected from the group consisting of:
the coupling reaction is carried out in an inert solvent such as THF, 2-methyl THF, dioxane, ether (C₁-C₄OC₁-C₄), C₁-C₅ alcohol or ester (C₁-C₄COOC₁-C₄));
the coupling reaction is carried out in the presence of Pd(0) or Pd(II) catalysts (such as Pd(PPh₃)₄, PdCl₂(PPh₃)₂, Pd(OAc)₂, PdCl₂(P(t-Bu)₂Ph)₂, Pd(dppf)₂Cl₂ or its methylene dichloride complex);
the hydrogenation reaction is carried out in the presence of Pt, Pd or Ni catalyst;
the hydrolysis reaction is carried out in the presence of proton acid and / or Lewis acid;
in the cyclization reaction the ammonium salt is selected from ammonium acetate, ammonium chloride, ammonium sulfate, ammonium phosphate, ammonium carbonate or ammonium hydrogen carbonate.

7. A method of preparation for a compound of formula 1-LDV, wherein, 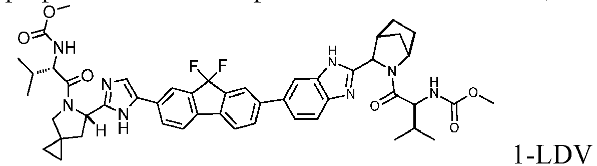
(7a) the method comprises steps of:
(i) reacting a compound of formula 10 with a compound of formula 11 to obtain a compound of formula 12; 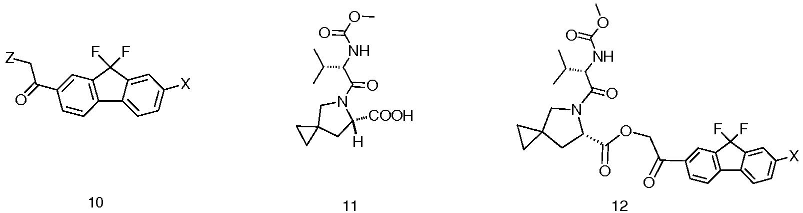
(ii) subjecting the compound of formula 12 with an ammonium salt to cyclization reaction to give a compound of formula 13; 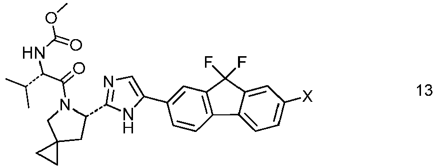
(iii) subjecting the compound of formula 13 with a compound of formula 17 to coupling reaction to give a compound of formula 18; 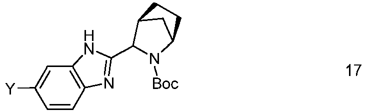 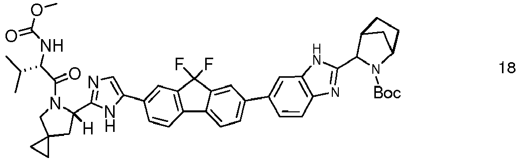
(iv) subjecting the compound of formula 18 to hydrolysis reaction to give a compound of formula 9; 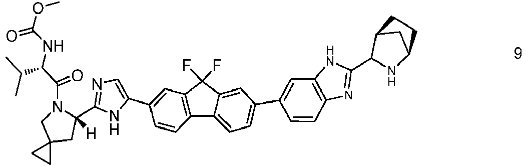
(v) reacting the compound of formula 9 with (S)-2-methoxycarbonylamino-3-methyl-butyric acid to produce a compound of formula 1-LDV;
wherein,
when X is halogen (Cl, Br or I), then Y is -B(OR₃)(OR₄) or -BF₃K; or when X is -B(OR₃)(OR₄) or -BF₃K, then Y is halogen (Cl, Br or I), wherein R₃ and R₄ are independently H or C1-C6 linear or branched alkyl, or two of them together form C2-C8 linear or branched alkylidene;
Z is halogen (chlorine, bromine or iodine);
or
(7b) the method comprises steps of:
(i) reacting a compound of formula 10 with a compound of formula 11 to obtain a compound of formula 12; 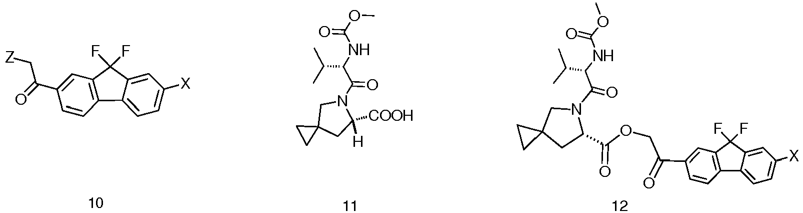
(ii) subjecting the compound of formula 12 with an ammonium salt to cyclization reaction to give a compound of formula 13; 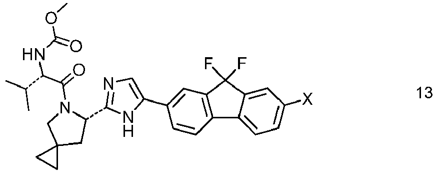
(iii) subjecting the compound of formula 13 to acylation reaction to produce a compound of formula 15; 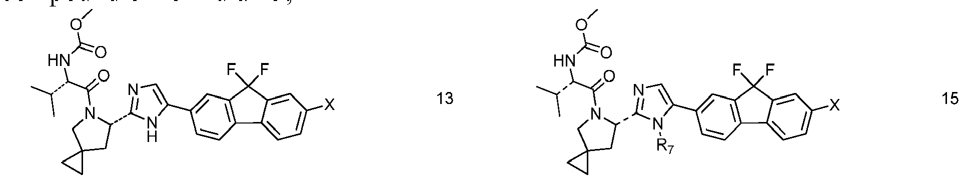 and subjecting a compound of formula 17 to acylation reaction to give a compound of formula 19; 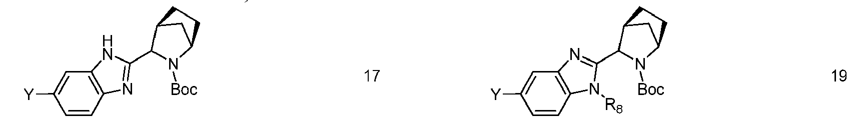
(iv) subjecting the compound of formula 15 with the compound of formula 19 to coupling reaction to obtain a mixture of a compound of formula 18' and a compound of formula 18; 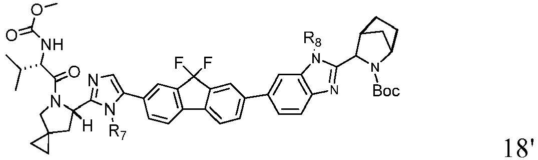 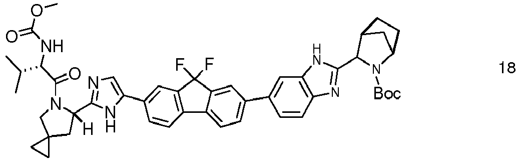
(v) subjecting the mixture of a compound of formula 18' and a compound of formula 18 to hydrolysis reaction to obtain a compound of formula 9; 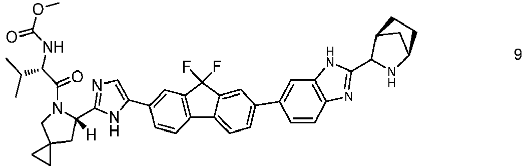
(vi) reacting the compound of formula 9 with (S)-2-methoxycarbonylamino-3-methyl-butyric acid to produce a compound of formula 1-LDV;
wherein,
when X is halogen (Cl, Br or I), then Y is -B(OR₃)(OR₄) or -BF₃K; or when X is -B(OR₃)(OR₄) or -BF₃K, then Y is halogen (Cl, Br or I), wherein R₃ and R₄ are independently H or C1-C6 linear or branched alkyl, or two of them together form C2-C8 linear or branched alkylidene;
Z is halogen (chlorine, bromine or iodine);
R₇ and R₈ are independently C1-C5 acyl, Cbz, Boc, or 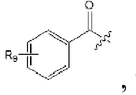 wherein, R₉ is a substituent at any position of benzene ring (ortho-, meta- or para-positionion), and the substituent is C1-C4 alkyl or halogen.

8. The method of claim 7, wherein, the method has one or more characteristics selected from the group consisting of:
the coupling reaction is carried out in an inert solvent such as THF, 2-methyl THF, dioxane, ether (C₁-C₄OC₁-C₄), C₁-C₅ alcohol or ester (C₁-C₄COOC₁-C₄);
the coupling reaction is carried out in the presence of Pd(0) or Pd(II) catalysts such as Pd(PPh₃)₄, PdCl₂(PPh₃)₂, Pd(OAc)₂, PdCl₂(P(t-Bu)₂Ph)₂, Pd(dppf)₂Cl₂ or its methylene dichloride complex;
the hydrolysis reaction is carried out in the presence of proton acid and / or Lewis acid.

9. The method of claim 7, wherein, in the cyclization reaction, the ammonium salt can be selected from the group consisting of ammonium acetate, ammonium chloride, ammonium sulfate, ammonium phosphate, ammonium carbonate and ammonium hydrogen carbonate.

10. Intermediate compounds for preparing Ledipasvir, 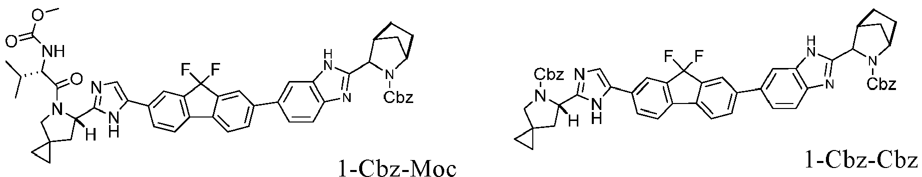 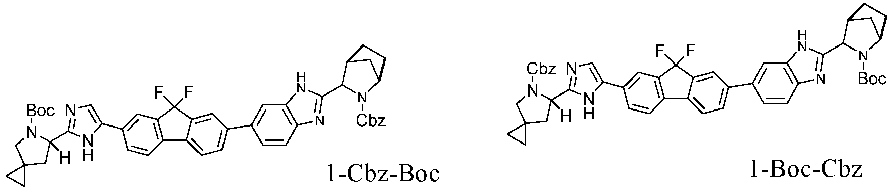 and 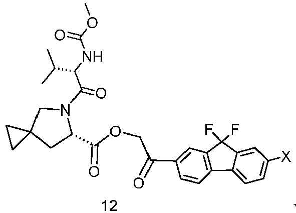 wherein, when X is halogen (Cl, Br or I), then Y is -B(OR₃)(OR₄) or -BF₃K; or when X is -B(OR₃)(OR₄) or -BF₃K, then Y is halogen (Cl, Br or I), wherein R₃ and R₄ are independently H or C1-C6 linear or branched alkyl, or two of them together form C2-C8 linear or branched alkylidene.
